# FASCICULE DE BREVET EUROPEEN

(11) **EP 0 740 942 B1**
(45) Date de publication et mention de la délivrance du brevet: **11.08.1999**
(21) Numéro de dépôt: 96390004.8
(22) Date de dépôt: 02.05.1996
(51) Int. Cl.: A61M 5/32, A61M 5/28, A61M 5/24, A61M 5/50

(54) **Procédé de fabrication d'un dispositif d'injection du type prérempli et dispositif d'injection réalisé**
Verfahren zur Herstellung einer vorgefüllten Injektionsvorrichtung und dadurch erhaltene Injektionsvorrichtung
Process of manufacturing a prefilled injection device and injection device produced thereby

(30) Priorité: 04.05.1995 FR 9505462
(43) Date de publication de la demande: 06.11.1996
(73) Titulaire: SANOFI Société Anonyme, 75008 Paris (FR)
(72) Inventeur: Brunel, Marc, 31000 Toulouse (FR)
(74) Mandataire: Cabinet BARRE LAFORGUE & associés

(56) Documents cités:
- EP-A- 0 191 508
- EP-A- 0 229 204
- EP-A- 0 325 049
- WO-A-92/17230
- WO-A-92/18183
- WO-A-93/00942
- WO-A-95/04565
- FR-A- 2 683 993
- FR-A- 2 698 546
- US-A- 3 066 670

## Description

L'invention concerne un procédé de fabrication d'un dispositif d'injection du type pré-rempli renfermant une dose de liquide à injecter. Elle s'étend à un dispositif d'injection réalisé selon ce procédé de fabrication.

A l'heure actuelle, toutes les seringues pré-remplies commercialisées possèdent un capuchon, destiné à protéger l'aiguille avant utilisation, qui est retiré en vue de l'injection, puis remis en place après injection en vue d'éviter les risques ultérieurs de piqûre avec l'aiguille souillée.

Une telle conception présente un inconvénient majeur résultant du fait que, lors du recapuchonnage de l'aiguille, le capuchon doit être présenté en regard de l'extrémité de cette aiguille. Cette obligation est la cause, en effet, d'accidents par piqûre relativement fréquents avec tous les risques que comportent de telles piqûres avec une aiguille souillée. De plus, de telles seringues ne présentent aucune sécurité visant à interdire leur réutilisation et doivent donc être l'objet de soins particuliers en vue de leur destruction.

Malgré ces inconvénients cruciaux, aucune solution rationnelle, tant du point de vue de la fabrication sur une échelle industrielle que du point de vue de la facilité d'utilisation des seringues, n'a été proposée à ce jour concernant les seringues pré-remplies, visant à équiper ces dernières d'un système de protection apte à éviter les risques de piqûre après usage et à interdire une utilisation ultérieure.

Un exemple d'un dispositif de l'art anterieur est decrit dans FR-A-2 698 546.

La présente invention vise à pallier ces inconvénients et a pour objectif essentiel de fournir un dispositif d'injection du type pré-rempli de faible prix de revient conçu pour éviter tout risque de piqûre après utilisation et pour interdire toute réutilisation ultérieure.

Un autre objectif de l'invention est de fournir un dispositif d'injection de moindre coût alliant les avantages précités et incorporant une seringue pré-remplie classique du type utilisé à l'heure actuelle.

Un autre objectif de l'invention est de fournir un dispositif d'injection permettant d'isoler, jusqu'au moment de l'injection, le liquide à injecter de l'aiguille d'injection.

Un autre objectif de l'invention est de fournir un dispositif d'injection possédant un volume "mort" très réduit et permettant donc d'injecter de très faibles doses de liquide.

Un autre objectif de l'invention est de fournir un dispositif d'injection particulièrement adapté pour permettre une injection nécessitant de maintenir les seringues dans une position inclinée par rapport au point d'injection.

A cet effet, l'invention vise un procédé de fabrication d'un dispositif d'injection pré-rempli tel que decrit dans la revendication 1.

Le procédé selon l'invention a donc consisté à réaliser un corps de seringue et un fourreau :
- dont l'assemblage est obtenu, en premier lieu, par un simple coulissement relatif desdits corps de seringue et fourreau, ce coulissement étant autorisé par la rétraction radiale de l'organe externe du corps de seringue,
- formant ensuite une structure indéformable grâce à l'introduction à l'intérieur du corps de seringue d'un récipient rigide conçu pour dilater radialement l'organe externe dudit corps de seringue et interdire toute déformation radiale ultérieure de ce dernier, garantissant ainsi un blocage irréversible du fourreau dans sa position avancée.

La fabrication d'un dispositif d'injection conforme à l'invention est donc réalisée en deux opérations très simples consistant chacune à amener un élément à coulisser longitudinalement relativement à un autre. Une telle fabrication peut donc être facilement automatisée et conduire à la production avec une cadence élevée de dispositifs d'injection présentant un faible prix de revient.

De plus, le dispositif d'injection réalisé est conçu pour éviter tout risque d'accident. En effet, il est doté d'un fourreau de protection amené à coulisser vers l'avant, une fois l'injection effectuée, sans risque aucun de piqûre car l'aiguille est recouverte en déplaçant ce fourreau en direction de l'extrémité antérieure du corps de seringue. En outre, une fois dans sa position avancée, ce fourreau est bloqué en translation relativement au corps de seringue, interdisant ainsi toute réutilisation du dispositif d'injection.

Il est à noter, par ailleurs, que le récipient rigide remplissant notamment la fonction de rigidification du corps de seringue, peut être mis en place soit dans une phase préalable au moment de la fabrication, soit seulement au moment de l'utilisation du dispositif d'injection.

Il convient de souligner que la demande de brevet internationale WO-95 04 565 décrit une seringue aspirante comportant un fourreau de protection, et dont à priori le fourreau est amené à coulisser le long de la seringue, en vue de l'assemblage de ces éléments.

Toutefois, la seringue est réalisée en un matériau plastique rigide et présente, en outre, une section rectangulaire, et de ce fait, au contraire de celle de l'invention, elle ne peut pas être déformée radialement.

Dans la pratique donc, le fourreau ne peut coulisser le long de la seringue qu'à condition soit de déformer par pincement cette seringue par une action mécanique ou manuelle, soit d'écarter les pattes postérieures 30, 31 du fourreau par une action mécanique ou manuelle.

De plus, il est à noter qu'il est spécifié dans ce brevet que :
- les fenêtres ménagées dans le fourreau et la seringue ont pour seule fonction de permettre de visualiser la carpule,
- cette carpule ne constitue pas un élément de rigidification destiné à assurer un blocage irréversible du fourreau dans sa position avancée, car du fait de la conception des organes de blocage, ce blocage irréversible est obtenu que la carpule soit ou non logée dans le corps de seringue.

En fait, même si ce brevet décrit un dispositif d'injection dont le procédé de fabrication présente certaines analogies avec celui de l'invention, le concept général à la base de l'invention diffère totalement de celui du procédé de ce brevet.

Selon une première variante préférentielle de mise en oeuvre, on réalise un corps de seringue comportant une paroi périphérique dotée d'au moins une fente longitudinale au droit de l'organe externe de blocage, apte à permettre les déformations radiales dudit organe de blocage.

Dans ce cas, les déformations radiales de l'organe externe du corps de seringue sont autorisées grâce à la présence d'au moins une fente, qui conduit cet organe externe à se rétracter en vue de permettre le passage de l'organe interne de blocage du fourreau, puis à se dilater lors de l'introduction du récipient rigide.

Selon une deuxième variante préférentielle de mise en oeuvre, on réalise un corps de seringue comportant un tronçon d'extrémité antérieure de forme tronconique de section décroissante en direction de l'extrémité antérieure dudit corps de seringue, et sur lequel est disposé l'organe externe de blocage, ledit tronçon tronconique étant doté longitudinalement de zones de faiblesse apte à permettre sa dilatation radiale.

Dans ce cas, l'extrémité antérieure du corps de seringue est préformée, lors du moulage dudit corps de seringue, de façon à conformer l'organe de blocage externe dans une position rétractée permettant le passage de l'organe interne de blocage du fourreau, la présence de zones de faiblesse permettant d'amener cet organe de blocage externe dans sa position dilatée radialement lors de l'introduction du récipient rigide.

Selon un mode de mise en oeuvre préférentiel visant à interdire tout coulissement intempestif du fourreau avant et durant l'injection, on solidarise les extrémités postérieures respectives du fourreau et du corps de seringue, dans la position d'injection de ces derniers, par des moyens de maintien aptes à pouvoir céder sous l'effet d'un effort de traction longitudinale exercée sur le fourreau et visant à le faire coulisser vers l'avant relativement au corps de seringue.

De tels moyens de maintien peuvent consister soit en des structures d'emboîtement conjuguées ménagées au niveau des extrémités postérieures respectives du fourreau et du corps de seringue, permettant de les solidariser de façon amovible. Ils peuvent également consister en au moins un point de solidarisation réalisé par tout moyen connu en soi tel que soudure à chaud, soudure par ultra-son, ...

Selon un premier mode de mise en oeuvre préférentiel :
- on réalise un corps de seringue doté d'une extrémité antérieure présentant la forme d'un manchon,
- on introduit dans le corps de seringue un récipient rigide constitué d'une seringue pré-remplie portant une aiguille d'injection, et présentant une portion antérieure de forme adaptée pour venir se loger dans le manchon du corps de seringue, de façon que l'aiguille d'injection s'étende dans le prolongement dudit corps de seringue.

Ce mode de mise en oeuvre permet de réaliser un dispositif d'injection comportant, en tant que récipient rigide, une seringue pré-remplie classique telle que les seringues actuelles réalisées en verre, tout en dotant, à moindre coût, lesdites seringues d'un système de protection garantissant contre tout risque d'accident après usage.

Selon un deuxième mode de mise en oeuvre préférentiel :
- on réalise un corps de seringue doté vers son extrémité antérieure d'un nez percé longitudinalement d'un alésage de section adaptée pour loger une aiguille d'injection,
- on introduit une aiguille d'injection dans l'alésage du nez du corps de seringue, de façon que cette dernière présente un tronçon actif d'injection dans le prolongement dudit corps de seringue, et un tronçon de percement débouchant à l'intérieur de celui-ci,
- on solidarise l'aiguille d'injection dans l'alésage du nez du corps de seringue,
- et on introduit dans le corps de seringue un récipient rigide pré-rempli doté d'un organe d'obturation apte à être percé par le tronçon de percement de l'aiguille d'injection.

Ce mode de mise en oeuvre permet de réaliser un dispositif d'injection comportant, en tant que récipient rigide, tout conteneur classique dont le système d'obturation peut être percé par une aiguille en vue de l'injection. Ainsi, un tel récipient peut être constitué d'une simple cartouche du type par exemple utilisé dans le domaine dentaire.

Selon un troisième mode de mise en oeuvre préférentiel :
- on réalise un corps de seringue doté d'une extrémité antérieure obturée par une paroi d'extrémité frontale percée d'un orifice, et comportant un alésage longitudinal, ménagé dans sa paroi périphérique, s'étendant entre ladite paroi frontale et un conduit radial interne débouchant à l'intérieur dudit corps de seringue, ce conduit radial étant ménagé à distance de la paroi frontale et étant juxtaposé longitudinalement à un épaulement délimitant, à l'intérieur du corps de seringue, une chambre d'extrémité, incorporant ledit conduit radial, de moindre section que la section interne courante dudit corps de seringue,
- on introduit une aiguille d'injection dans l'alésage longitudinal de façon que cette dernière possède un tronçon actif d'injection dans le prolongement de la paroi frontale du corps de seringue,
- on solidarise l'aiguille d'injection dans l'alésage longitudinal du corps de seringue,
- et on introduit, à l'intérieur du corps de seringue, un tube rigide pré-rempli de section adaptée pour venir en butée contre l'épaulement interne dudit corps de seringue, ledit tube rigide étant obturé, vers chacune de ses extrémités, par un bouchon coulissant, dit respectivement antérieur et postérieur, le bouchon antérieur présentant une forme adaptée pour venir se loger dans la chambre d'extrémité du corps de seringue, lors du coulissement du bouchon postérieur, dans une position où il vient buter contre la paroi frontale et dégage le conduit radial.

Ce mode de mise en oeuvre permet de réaliser des dispositifs d'injection possédant un volume "mort" très réduit, particulièrement adaptés pour l'injection de très faibles doses de liquide.

En outre, du fait de la position de l'aiguille d'injection disposée latéralement par rapport au corps de seringue, et moyennant une orientation correcte du biseau du tronçon actif d'injection de cette dernière, ce dispositif d'injection est particulièrement adapté pour permettre une injection (par exemple dans le ventre) nécessitant de maintenir l'aiguille d'injection dans une position inclinée par rapport au point d'injection.

Par ailleurs, pour chacune des applications précitées, on encapuchonne avantageusement, de façon classique, l'extrémité avant du corps de seringue en vue de protéger l'aiguille avant utilisation et à éviter tout risque de piqûre avec cette dernière.

L'invention s'étend à un dispositif d'injection du type pré-rempli tel que decrit dans la revendication 9.

Selon une première variante de réalisation préférentielle, le corps de seringue comporte un tronçon d'extrémité antérieure de section décroissante en direction de l'extrémité antérieure dudit corps de seringue, et sur lequel est disposé l'organe externe de blocage, ledit tronçon tronconique étant doté longitudinalement de zones de faiblesse aptes à permettre sa dilatation radiale.

Selon une deuxième variante de réalisation préférentielle, le corps de seringue comporte une paroi périphérique dotée d'au moins une fente longitudinale s'étendant au droit de l'organe externe de blocage, apte à permettre les déformations radiales dudit organe externe de blocage.

Dans ce cas, en outre, le corps de seringue comporte avantageusement une paroi périphérique dotée de deux fentes longitudinales diamétralement opposées. Cette disposition des lumières longitudinales est, en effet, avantageuse lors de la fabrication par moulage plastique des corps de seringue.

Selon une autre caractéristique de l'invention :
- l'organe de blocage de l'un des éléments, corps de seringue ou fourreau présente la forme d'un bossage annulaire comportant une rainure annulaire et des crans, disposés de part et d'autre de cette dernière,
- l'organe de blocage de l'autre élément, fourreau ou corps de seringue, est constitué d'une nervure annulaire délimitant une section conjuguée de la section du fond de la rainure du bossage du premier élément.

De plus, les crans de l'organe de blocage ménagé sur l'un des éléments, corps de seringue ou fourreau, et situés vers l'extrémité dudit élément, présentent avantageusement une épaisseur supérieure à celle des deuxièmes crans délimitant la rainure dudit organe de blocage.

De ce fait, les premiers crans provoquent une déformation radiale du corps de seringue qui facilite le passage des deuxièmes crans et garantit un emmanchement aisé du fourreau sur ledit corps de seringue.

En outre, selon une autre caractéristique de l'invention, les deuxièmes crans de l'organe de blocage ménagé sur un des éléments, corps de seringue ou fourreau, présentent une face frontale s'étendant selon une corde dudit élément, adaptée pour obtenir au niveau desdits crans un périmètre au moins égal à celui de l'autre élément au droit de la nervure annulaire de ce dernier.

Ainsi, le fourreau est simplement déformé triangulairement lors du passage des deuxièmes crans au droit de la nervure annulaire de blocage, sans avoir à subir de dilatation radiale en vue de ce passage, ce qui facilite l'amenée dudit fourreau dans sa position avancée de verrouillage, après utilisation.

Selon une autre caractéristique de l'invention, le corps de seringue présente un diamètre interne sensiblement supérieur à celui du diamètre externe du récipient rigide, et comporte des nervures internes longitudinales au droit de l'organe interne de blocage dudit corps de seringue, déterminant un diamètre conjugué du diamètre externe du récipient rigide.

Cette disposition autorise de mettre une étiquette autour du récipient rigide et d'obtenir, malgré la surépaisseur que forme cette étiquette, un contact intime entre le corps de seringue et le récipient rigide lors de l'introduction de ce dernier.

Selon un premier mode de réalisation préférentiel de ce dispositif d'injection :
- le corps de seringue comporte une extrémité antérieure présentant la forme d'un manchon,
- le récipient rigide pré-rempli est constitué d'une seringue pré-remplie portant une aiguille d'injection, et présentant une portion antérieure de forme adaptée pour venir se loger dans le manchon du corps de seringue, de façon que l'aiguille d'injection s'étende dans le prolongement dudit corps de seringue.

En outre, lorsque la seringue pré-remplie est classiquement dotée d'une extrémité postérieure comportant une collerette de prise digitale, le corps de seringue comprend alors préférentiellement, au droit de son extrémité postérieure, des crans radiaux aptes à loger ladite collerette et à assurer le blocage en translation de ladite seringue pré-remplie.

De plus, les crans radiaux sont préférentiellement ménagés dans une bague en forme de coupelle de diamètre supérieur à celui du corps de seringue, disposée dans le prolongement postérieur dudit corps de seringue, ladite bague étant adaptée pour servir de butée frontale de l'extrémité postérieure du fourreau.

Par ailleurs, le dispositif d'injection comprend également, de façon avantageuse un jonc annulaire déformable agencé pour être sollicité par la collerette de la seringue, de façon à assurer un blocage sans jeu de cette dernière dans les crans radiaux.

Un tel jonc a pour avantage d'assurer un blocage parfait de la seringue et ce, malgré les variations d'épaisseur de la collerette de cette dernière résultant des tolérances de fabrication.

Selon un deuxième mode de réalisation préférentiel de ce dispositif d'injection :
- le corps de seringue est doté, vers son extrémité antérieure, d'un nez percé longitudinalement d'un alésage logeant une aiguille d'injection de façon que cette dernière présente un tronçon actif d'injection dans le prolongement dudit corps de seringue, et un tronçon de percement débouchant à l'intérieur de celui-ci,
- le récipient rigide pré-rempli est constitué d'une cartouche dotée d'un organe d'obturation apte à être percé par le tronçon de percement de l'aiguille d'injection.

De plus, l'organe d'obturation de la cartouche consiste alors, préférentiellement en un bouchon apte à être percé, disposé, dans une phase initiale, de façon à se trouver à distance de l'extrémité du tronçon de percement de l'aiguille d'injection, ladite cartouche étant obturée vers son autre extrémité, par un deuxième bouchon faisant office de piston.

Une telle disposition permet d'isoler, jusqu'au moment de l'injection, le liquide à injecter de l'aiguille d'injection. De ce fait, et en premier lieu, ce liquide n'est au contact ni de l'aiguille ni de la colle utilisée pour solidariser cette dernière, et ne risque donc pas d'être pollué. De plus l'aiguille n'a pas à être obturée, tel que de façon classique en la piquant dans le fond d'un capuchon, et ses qualités de pénétration sont donc totalement préservées.

Selon une autre caractéristique de l'invention visant ce deuxième mode de réalisation préférentiel, le corps de seringue comprend, au droit de son extrémité postérieure, une nervure interne apte à assurer le blocage en translation de la cartouche introduite dans ledit corps de seringue.

Selon un troisième mode de réalisation préférentiel de ce dispositif d'injection :
- le corps de seringue est doté d'une extrémité antérieure obturée par une paroi d'extrémité frontale percée d'un orifice, et comporte un alésage longitudinal ménagé dans sa paroi périphérique, logeant une aiguille d'injection de façon que cette dernière présente un tronçon actif d'injection dans le prolongement de la paroi frontale, ledit alésage s'étendant entre ladite paroi frontale et un conduit radial interne débouchant à l'intérieur dudit corps de seringue, ce conduit radial étant ménagé à distance de la paroi frontale et étant juxtaposé longitudinalement à un épaulement délimitant, à l'intérieur du corps de seringue, une chambre d'extrémité incorporant ledit conduit radial, de moindre section que la section interne courante dudit corps de seringue,
- le récipient rigide pré-rempli est constitué d'une cartouche de section adaptée pour venir en butée contre l'épaulement interne du corps de seringue, ladite cartouche étant obturée, vers chacune de ses extrémités, par un bouchon coulissant, dit respectivement antérieur et postérieur, le bouchon postérieur faisant office de piston, et le bouchon antérieur présentant une forme adaptée pour venir se loger dans la chambre d'extrémité du corps de seringue, lors de l'actionnement du bouchon postérieur, dans une position où il vient en butée contre la paroi frontale et dégage l'accès au conduit radial.

Un tel dispositif d'injection possède un volume "mort" très réduit et se trouve particulièrement adapté pour l'injection de faibles doses de liquide. De plus, il permet d'isoler jusqu'au moment de l'injection le liquide à injecter de l'aiguille d'injection.

Par ailleurs, le corps de seringue comprend alors, préférentiellement, au droit de son extrémité postérieure, une nervure interne apte à assurer le blocage en translation de la cartouche introduite dans ledit corps de seringue.

D'autres caractéristiques, buts et avantages de l'invention ressortiront de la description détaillée qui suit en référence aux dessins annexés, qui en représentent à titre d'exemples non limitatifs cinq modes de réalisation préférentiels. Sur ces dessins qui font partie intégrante de la présente description :
- la figure 1 est une coupe longitudinale par un plan axial d'un premier mode de réalisation de dispositif d'injection conforme à l'invention, représenté avant utilisation,
- la figure 2 est une coupe longitudinale par un plan axial représentant ce dispositif d'injection après utilisation,
- la figure 3 est une coupe longitudinale par un plan axial du corps de seringue de ce dispositif d'injection,
- la figure 4 est une vue frontale de l'extrémité postérieure de ce corps de seringue,
- la figure 5 est une vue frontale de l'extrémité antérieure de ce corps de seringue,
- la figure 6 est une coupe longitudinale par un plan axial du fourreau de ce dispositif d'injection,
- la figure 7 est une vue frontale de l'extrémité postérieure de ce fourreau,
- les figures 8 à 11 représentent les différents stades de fabrication de ce dispositif d'injection,
- la figure 12a représente un deuxième mode de réalisation du dispositif d'injection conforme à l'invention avant assemblage du fourreau et du corps de seringue, sur laquelle le fourreau est représenté en coupe longitudinale par un plan axial, et le corps de seringue en vue longitudinale,
- la figure 12b est une vue frontale de l'extrémité antérieure du corps de seringue de ce dispositif d'injection, dans l'état rétracté radialement de ce dernier,
- les figures 13a et 13b sont des représentations de ce dispositif d'injection, correspondant aux figures 12a et 12b, après assemblage du corps de seringue et du fourreau et introduction du récipient rigide,
- la figure 14 est une coupe longitudinale par un plan axial d'un troisième mode de réalisation d'un dispositif d'injection conforme à l'invention, représenté avant utilisation,
- la figure 15 est une coupe longitudinale par un plan axial représentant ce dispositif d'injection après utilisation,
- la figure 16 est une coupe transversale au droit d'un plan A de ce dispositif d'injection, représentant le fourreau en fin de coulissement,
- la figure 17 est une coupe longitudinale par un plan axial du corps de seringue de ce dispositif d'injection,
- la figure 18 est une coupe longitudinale par un plan axial d'un quatrième mode de réalisation de dispositif d'injection conforme à l'invention, représenté avant utilisation,
- la figure 19 est une coupe longitudinale par un plan axial C du corps de seringue de ce dispositif d'injection,
- la figure 20 est une coupe longitudinale par un plan axial d'un cinquième mode de réalisation de dispositif d'injection conforme à l'invention, représenté avant utilisation,
- la figure 21 en est une coupe transversale par un plan D,
- et la figure 22 est une coupe longitudinale partielle par un plan axial de ce dispositif d'injection, représenté en cours d'injection.

Les dispositifs d'injection représentés à titre d'exemples aux figures sont tous constitués de trois éléments principaux consistant en un corps de seringue adapté pour posséder une faculté de déformation radiale, un fourreau adapté pour être monté autour du corps de seringue et destiné à protéger l'aiguille après injection, et un récipient rigide pré-rempli introduit dans le corps de seringue, soit lors de la fabrication soit seulement avant injection, et destiné notamment à conférer un caractère indéformable au corps de seringue.

Le corps de seringue et le fourreau de ces dispositifs sont, en outre, réalisés en un matériau plastique déformable tel que du styrène butadienne.

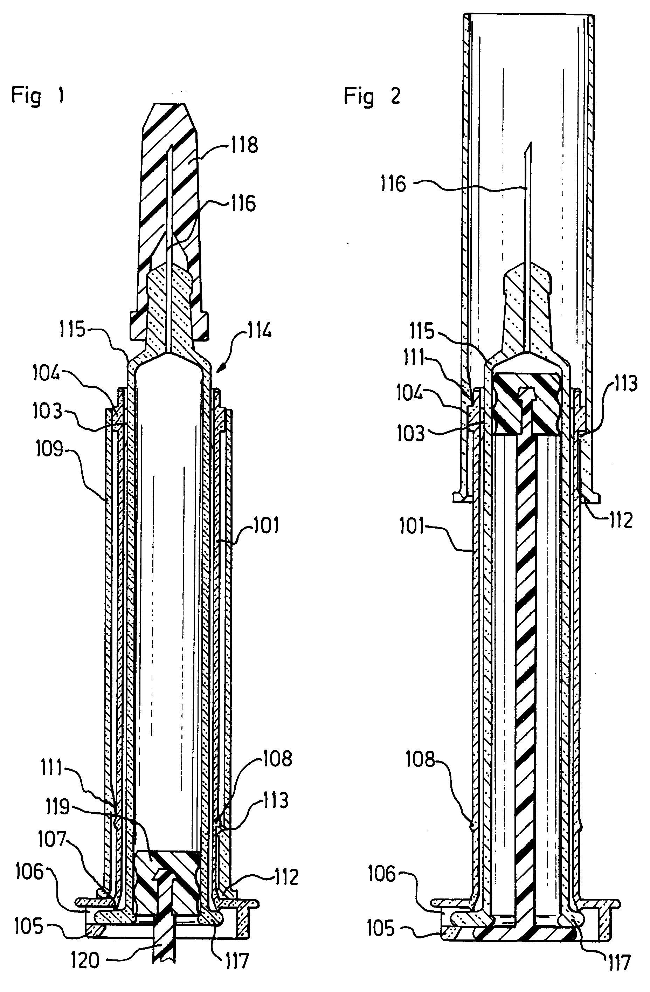

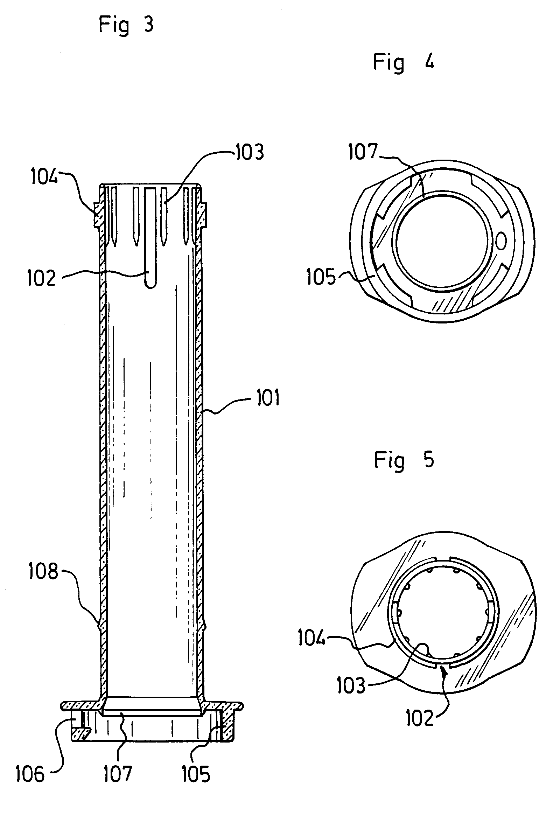

En premier lieu, le dispositif d'injection représenté aux figures 1 à 7, comprend un corps de seringue tubulaire 101 représenté aux figures 3 à 5 dont la paroi périphérique est dotée de deux fentes longitudinales telles que 102 diamétralement opposées.

Chacune de ces fentes 102 est ménagée au niveau du tronçon antérieur du corps de seringue 101, présente une longueur au moins égale à l'organe de blocage décrit ci-après dudit corps de seringue 101, et s'étend au moins en regard dudit organe de blocage. De plus, elles ne débouchent pas au droit de l'extrémité antérieure du corps de seringue 101 de façon à éviter que lesdits corps de seringues ne viennent s'imbriquer les uns dans les autres lors de leur transport sur une chaîne automatisée.

Ce corps de seringue 101 comprend, en outre, des nervures longitudinales internes telles que 103 s'étendant au droit de son tronçon d'extrémité antérieure et uniformément réparties autour de l'axe de symétrie dudit corps de seringue.

De plus, ce corps de seringue 101 comprend, dans sa portion d'extrémité antérieure, un organe externe de blocage consistant en une nervure annulaire externe 104.

Côté extrémité postérieure, ce corps de seringue 101 est prolongé par une bague 105 en forme de coupelle de diamètre supérieur à celui dudit corps de seringue formant l'organe de prise digitale dudit corps de seringue, et dans laquelle sont ménagés des crans radiaux tels que 106.

De plus, un jonc annulaire déformable 107 est disposé à l'intérieur de cette bague 105 directement dans le prolongement du corps de seringue 101.

Enfin, ce corps de seringue 101 comprend une nervure annulaire externe 108 ménagée à une distance de son extrémité postérieure prédéterminée tel qu'explicité ci-après.

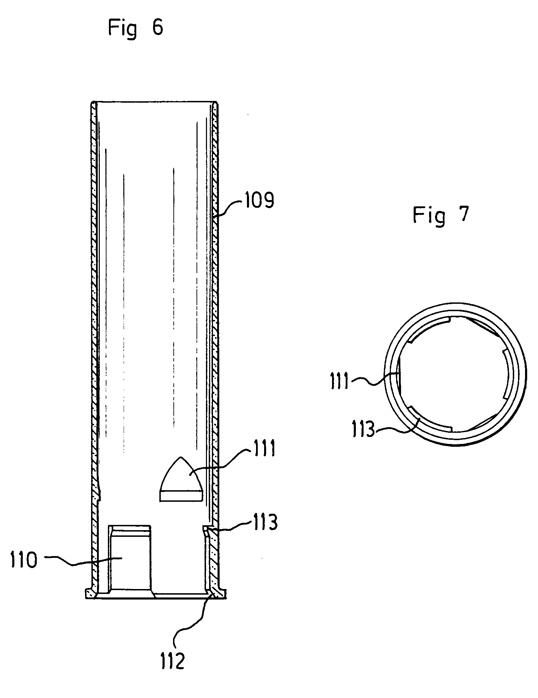

Tel que représenté aux figures 6 et 7, le fourreau 109 de ce dispositif d'injection consiste en un fourreau cylindrique de diamètre interne conjugué du diamètre externe de la nervure annulaire de blocage 104 du corps de seringue 101.

Ce fourreau 109 comprend des moyens de blocage interne disposés au niveau de son extrémité postérieure et destinés à coopérer avec la nervure externe 104 du corps de seringue 101.

Ces moyens de blocage comprennent deux séries de crans tels que 110 , 111 décalées axialement de façon à délimiter, entre lesdites séries de crans, un espace annulaire apte à loger la nervure externe 104 du corps de seringue 101.

De plus, les crans 110, 111 de chaque série, sont uniformément répartis autour de l'axe longitudinal du fourreau 109, et les crans de chaque série sont décalés par rapport à ceux de l'autre série en vue de permettre le démoulage du fourreau 109.

En premier lieu, les crans postérieurs 110 s'étendent à partir de l'extrémité postérieure du fourreau 109 et présentent la forme de secteurs annulaires en saillie par rapport à la face interne dudit fourreau.

De plus, ces crans postérieurs 110 présentent une extrémité postérieure chanfreinée 112, et comportent une nervure annulaire 113 formant une surépaisseur au droit de leur extrémité antérieure.

Les crans 111 de l'autre série sont ménagés, tel que représenté à la figure 7, de façon que leur face frontale s'étende selon une corde de la face interne du fourreau. De plus, ils présentent une épaisseur décroissante en direction de l'extrémité antérieure du fourneau 109, ce qui leur confère, vu de face et tel que représenté à la figure 6, la forme d'une ogive.

En outre, ces crans 111 présentent une épaisseur maximale inférieure à l'épaisseur des crans postérieur 110 au droit de la nervure annulaire antérieure 113 de ces derniers.

La seringue de ce premier mode de réalisation est, quant à elle, une seringue pré-remplie 114 de type traditionnel, telle que par exemple réalisée en verre, comportant de façon classique :
- un nez antérieur 115 sur lequel est monté une aiguille d'injection 116,
- une collerette externe 117 au droit de son extrémité postérieure, adaptée pour venir se loger dans les crans radiaux 106 du corps de seringue 101, de façon à assurer un blocage en translation de ladite seringue,
- un capuchon 118 de protection de l'aiguille d'injection 116 avant utilisation, dans le fond duquel l'extrémité de ladite aiguille est plantée afin d'éviter tout écoulement de liquide,
- et un bouchon 119 faisant office de piston, associé à une tige de piston 120.

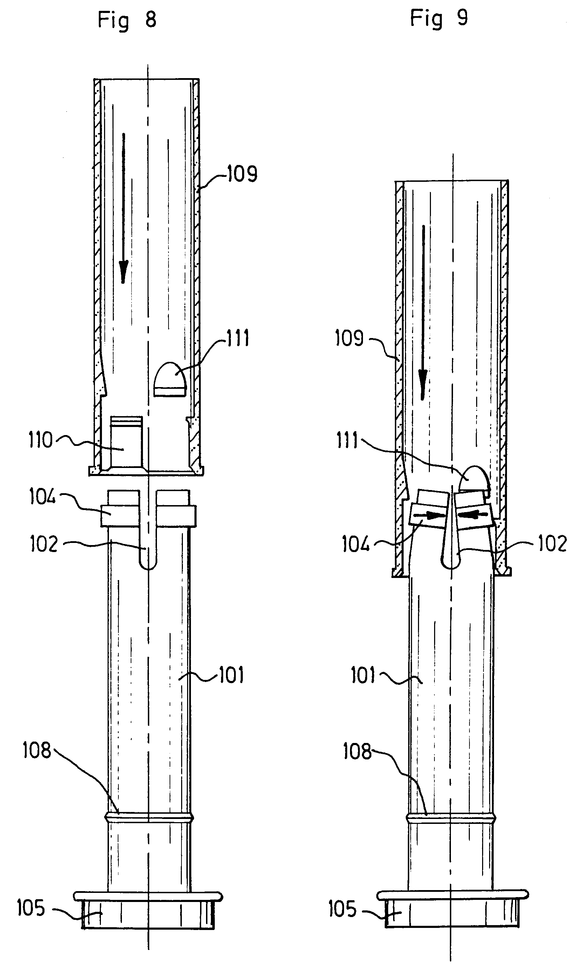

Les étapes de montage d'un tel dispositif d'injection sont représentées aux figures 8 à 11.

En premier lieu, le fourreau 109 est présenté en regard de l'extrémité antérieure du corps de seringue 101 (figure 8), puis emmanché sur ce dernier.

Lors de cet emmanchement, les crans postérieurs 110 du fourreau 109 provoquent une rétraction radiale de la nervure annulaire 104 du corps de seringue 101, grâce à la présence des fentes 102 (figure 9).

En outre, du fait que ces crans postérieurs 110 présentent une épaisseur supérieure à celle des autres crans 111, cette déformation radiale est telle que le passage de ces crans 111 au droit de la nervure annulaire 104 ne pose pas de problème.

Après usage, ce passage des crans 111 au droit de la nervure annulaire 104 est, en outre, facilité du fait de la forme de ces crans 111 qui conduisent à obtenir une simple déformation triangulaire du fourreau 109 sans augmentation du diamètre de ce dernier lors de ce passage.

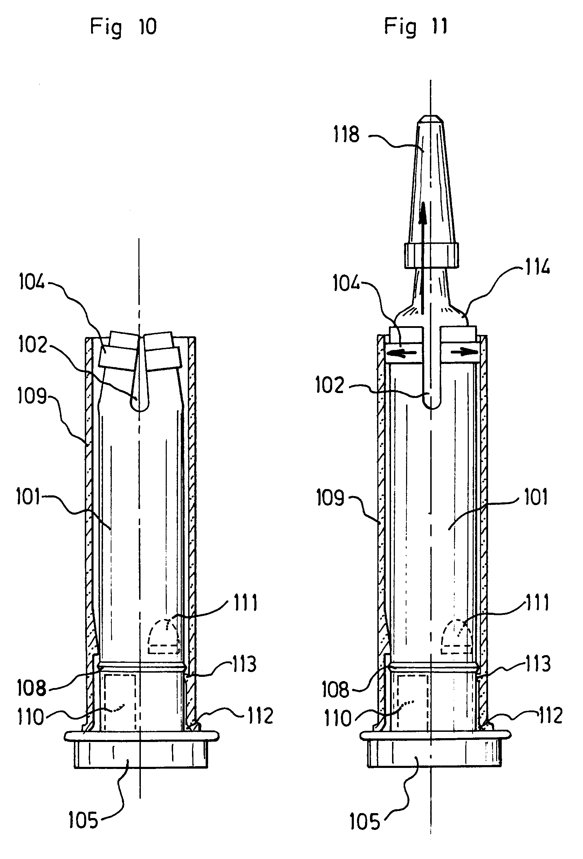

En fin d'emmanchement, et tel que représenté à la figure 10, le corps de seringue 101 se trouve entièrement logé à l'intérieur du fourreau 109 dont l'extrémité postérieure est en butée frontale contre la bague 105 dudit corps de seringue.

De plus, dans cette position, les crans postérieurs 110 du fourreau 109 coopèrent avec la nervure annulaire 108 du corps de seringue 101, conduisant à un blocage relatif en translation desdits éléments, sauf à exercer un effort de traction suffisant, pour amener les crans postérieurs 110 à franchir la nervure 108.

En outre, tel que représenté de façon volontairement accentuée à la figure 10, l'organe de blocage 104 du corps de seringue 101 conserve une déformation radiale rémanente résultant de la nature du matériau constituant ce corps de seringue 101.

La dernière étape consiste à introduire la seringue pré-remplie 114 à l'intérieur du corps de seringue 101. Lors de cette introduction, du fait de la présence des nervures internes 103, la seringue pré-remplie 114 tend à dilater radialement la nervure annulaire 104 du corps de seringue 101.

En fin d'introduction, la collerette 117 de la seringue 114 vient se loger dans les crans radiaux 106 dans lesquels elle se trouve bloquée sans jeu grâce à la présence du jonc 107.

Le corps de seringue 101 et le fourreau 109 forment alors une structure indéformable garantissant ainsi un blocage du fourreau 109 lorsque celui-ci est amené dans sa position avancée, après utilisation du dispositif d'injection.

De plus, il est à noter que lorsque le fourreau 109 est amené à coulisser vers l'avant, le fait que les crans postérieurs 110 présentent une nervure antérieure 113, permet d'éviter que lesdits crans ne viennent frotter contre la nervure 108 du corps de seringue 101 et s'opposer à cette translation.

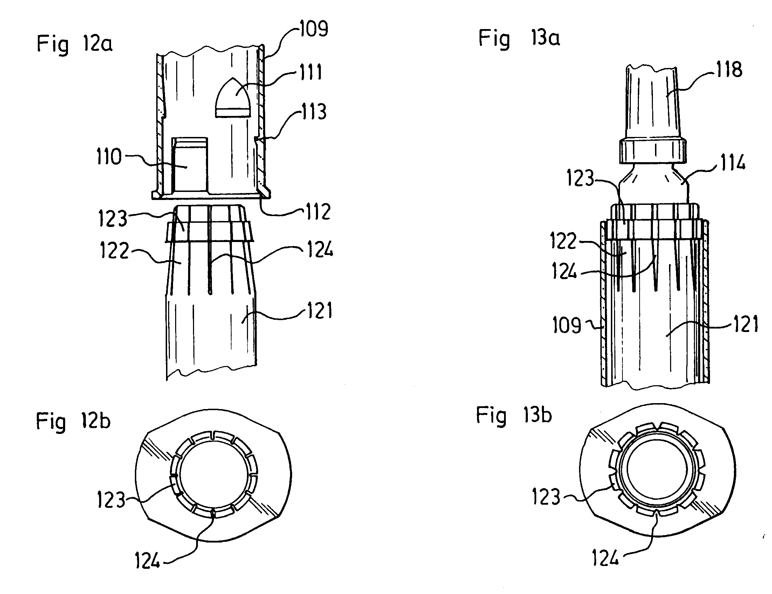

Les figures 12a, 12b, 13a, 13b, représentent un deuxième mode de réalisation d'un dispositif d'injection conforme à l'invention comportant un fourreau 109 et une seringue pré-remplie 114 similaires à ceux ci-dessus décrits, et un corps de seringue 121 dont la partie postérieure est également identique à celle du corps de seringue 101 précédemment décrit, et n'est donc pas représentée.

La particularité de ce corps de seringue 121 réside dans le fait qu'il comporte un tronçon antérieur 122 sur lequel est ménagé une nervure annulaire externe de blocage 123, préformé de façon à présenter une forme tronconique de section décroissante en direction de l'extrémité antérieure dudit corps de seringue.

Ce tronçon tronconique 122 comporte, en outre, des zones de faiblesse 124 consistant en des lignes longitudinales de moindre épaisseur réparties autour de l'axe du corps de seringue 121.

Tel que représenté à la figure 12a, une telle conception permet d'emmancher aisément le fourreau 109, du fait que la nervure annulaire 123 du corps de seringue 121 se trouve dans une position rétractée radialement autorisant le passage des crans 110 et 111.

De plus, tel que représenté à la figure 13a, l'introduction ultérieure de la seringue 114 à l'intérieur du corps de seringue 121, une fois le fourreau 109 mis en place, conduit à dilater radialement le tronçon tronconique 122 et donc la nervure annulaire 123 du fait de la présence des zones de faiblesse 124.

De ce fait, on obtient également un dispositif d'injection présentant les mêmes avantages que celui précédemment décrit

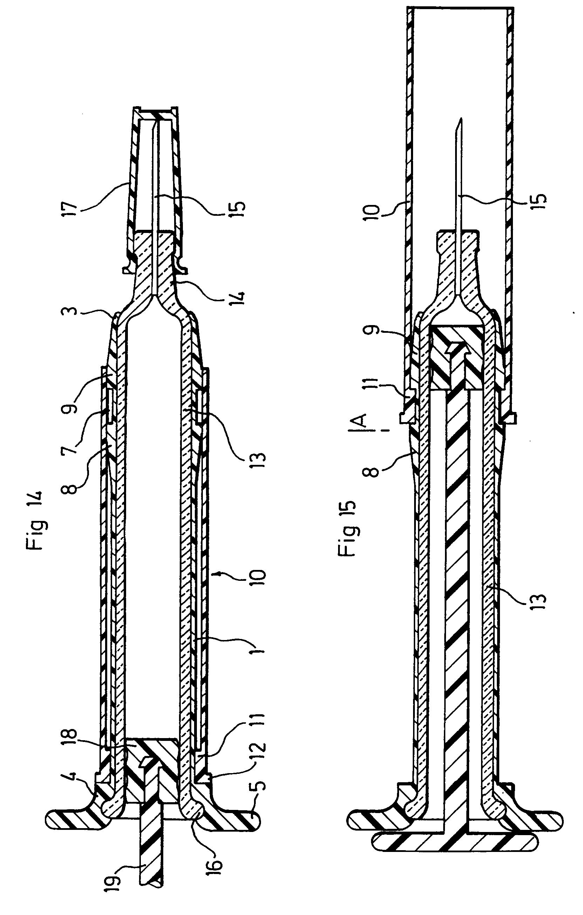

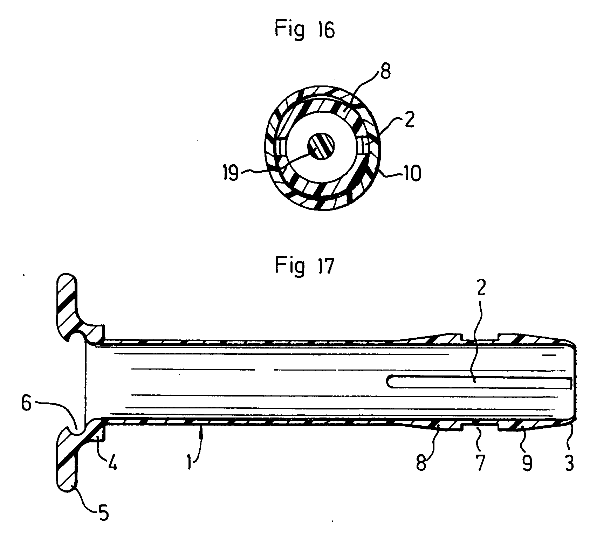

Le mode de réalisation de dispositif d'injection conforme à l'invention représenté aux figures 14 à 16 est similaire dans son principe à celui du premier mode de réalisation. Il comprend donc un corps de seringue tubulaire 1, représenté à la figure 17, dont la paroi périphérique est dotée de deux fentes longitudinales telles que 2 diamétralement opposées.

Vers son extrémité antérieure, ce corps de seringue 1 forme un manchon 3 de section décroissante adaptée pour former un siège de maintien de la seringue pré-remplie.

Vers son extrémité postérieure, ce corps de seringue 1 comporte un épaulement annulaire externe 4 formant une face de butée frontale, et deux ailettes de prise digitale, telles que 5, s'étendant symétriquement de part et d'autre de la portion arrière de cet épaulement. En outre, cet épaulement 4 comporte, au droit des ailettes 5 une encoche interne 6 de section arrondie, de blocage de la seringue tel que décrit ci-après.

Ce corps de seringue 1 comprend enfin, dans sa portion d'extrémité antérieure, un organe externe de blocage présentant la forme d'un bossage annulaire interrompu au droit de chaque fente 2, comportant une rainure annulaire 7 et deux rampes inclinées 8, 9 d'accès à ladite rainure s'étendant longitudinalement de part et d'autre de cette dernière.

Le fourreau de ce troisième mode de réalisation consiste en un fourreau cylindrique 10 de diamètre interne conjugué de la section externe du bossage 7-9 du corps de seringue.

Ce fourreau 10 comporte, vers son extrémité postérieure, une nervure annulaire interne 11, délimitant une section interne conjuguée de la section externe courante du corps de seringue.

Ce fourreau 10 comporte, enfin, au droit de son extrémité postérieure un épaulement externe 12 formant, avec la face d'extrémité postérieure de la nervure 11, une face de butée frontale conjuguée de celle de l'épaulement 4 du corps de seringue 1.

La seringue de ce premier mode de réalisation est, quant à elle, une seringue pré-remplie 13 de type traditionnel, comportant tel que précédemment :
- un nez antérieur 14 sur lequel est montée une aiguille d'injection 15, et formant un col dont la base est adaptée pour coopérer avec la surface interne du manchon 3 du corps de seringue,
- une collerette externe 16 au droit de son extrémité postérieure, adaptée pour venir se loger dans l'encoche 6 du corps de seringue 1 de façon à assurer un blocage en translation de ladite seringue,
- un capuchon 17 de protection de l'aiguille d'injection 15,
- et un bouchon 18 faisant office de piston, associé à une tige de piston 19.

Tel que précédemment, le montage d'un tel dispositif d'injection consiste à amener le fourreau 10 à coulisser longitudinalement le long du corps de seringue jusqu'à amener les épaulements postérieurs 4, 12 respectifs de ces derniers en butée frontale.

Une fois cette mise en place effectuée, les extrémités postérieures du fourreau 10 et du corps de seringue 1 sont solidarisées, au droit des faces de jonction des épaulements 4, 12, par au moins un point de solidarisation, réalisé par soudure à chaud, soudure à ultra-sons, ... apte à pouvoir céder sous l'effet d'un effort de traction longitudinale exercée sur ledit fourreau visant à le faire coulisser vers l'avant.

Enfin, la seringue pré-remplie est introduite dans le corps de seringue 1 dans lequel elle vient se bloquer grâce à la coopération de la collerette 16 et de l'encoche 6, assurant, une fois introduite, la rigidification dudit corps de seringue et formant avec ce dernier une structure indéformable.

Une fois ce montage effectué, l'injection est réalisée de façon classique après retrait du capuchon de protection 17.

Après injection, une traction exercée sur le fourreau 10 permet de faire coulisser ce dernier vers l'avant jusqu'à amener la nervure 11 dudit fourreau à l'intérieur de la rainure 7 du corps de seringue, position dans laquelle l'aiguille d'injection 15 est protégée par le fourreau.

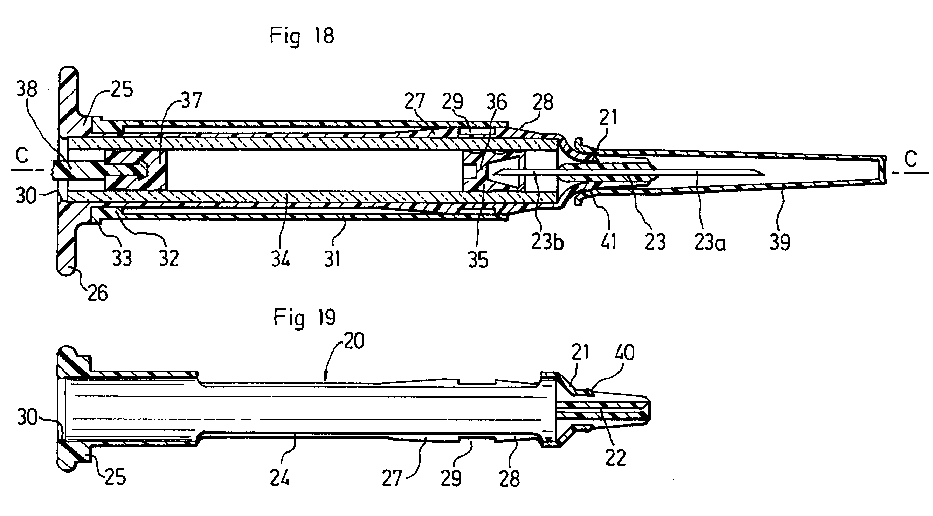

Le dispositif d'injection représenté à la figure 18 comporte un corps de seringue tubulaire 20, représenté à la figure 19, doté vers son extrémité antérieure d'un nez 21 percé longitudinalement d'un alésage 22 à l'intérieur duquel est introduite une aiguille d'injection 23 solidarisée à l'intérieur dudit alésage par tout moyen connu tel que de la colle, de façon à présenter un tronçon actif d'injection 23a dans le prolongement du corps de seringue 1 et un tronçon de percement 23b débouchant à l'intérieur de celui-ci.

Comme celui décrit en référence aux figures 14 à 16, ce corps de seringue 20 comprend deux fentes longitudinales 24, un épaulement postérieur externe 25 de part et d'autre duquel s'étendent deux ailettes de prise digitale telles que 26, et un bossage formé de deux rampes 27, 28 s'étendant longitudinalement de part et d'autre d'une rainure annulaire 29.

En outre, ce corps de seringue 20 comporte, au droit de l'extrémité postérieure de l'épaulement 25, une nervure interne annulaire 30 destinée au blocage en translation du récipient rigide pré-rempli décrit ci-après.

Le fourreau 31 de ce dispositif d'injection est quant à lui strictement identique à celui décrit précédemment et comprend donc une nervure interne postérieure 32 agencée pour coulisser le long du corps de seringue 20, et un épaulement postérieur 33 de butée contre l'épaulement 25 dudit corps de seringue.

Le récipient rigide de ce dispositif d'injection est constitué d'une cartouche 34 se présentant sous la forme d'un tube cylindrique de dimensions adaptées pour venir se loger dans le corps de seringue dans lequel elle se trouve bloquée par la nervure annulaire 30 de ce dernier.

Cette cartouche 34 est obturée, au niveau de son extrémité antérieure, par un bouchon 35 évidé intérieurement de façon à former une membrane 36 apte à être percée par le tronçon de percement 23b de l'aiguille d'injection 23, et, au niveau de son extrémité postérieure, par un deuxième bouchon 37 faisant office de piston associé à une tige de piston 38.

En outre, ces deux bouchons 35, 37 sont disposés de façon que la membrane 36 du bouchon antérieur 35 se trouve à distance de l'extrémité arrière du tronçon de percement 23b de l'aiguille d'injection 23, tant que le bouchon postérieur 37 n'est pas sollicité. De ce fait, le liquide renfermé dans la cartouche 34 se trouve isolé de l'aiguille d'injection 23 jusqu'au moment de l'injection.

En dernier lieu, ce dispositif d'injection comporte un capuchon 39 apte à loger le tronçon actif 23a de l'aiguille d'injection 23, ledit capuchon et le nez 21 du corps de seringue comportant des organes d'encliquetage conjugués aptes à permettre de les solidariser de façon amovible.

En l'exemple, ces organes d'encliquetage consistent en une nervure annulaire externe 40 ménagée sur le nez 21, apte à coopérer avec une gorge annulaire interne 41 du capuchon 39.

Les principes de montage et d'utilisation de ce dispositif d'injection sont strictement identiques à ceux des dispositifs d'injection décrits précédemment.

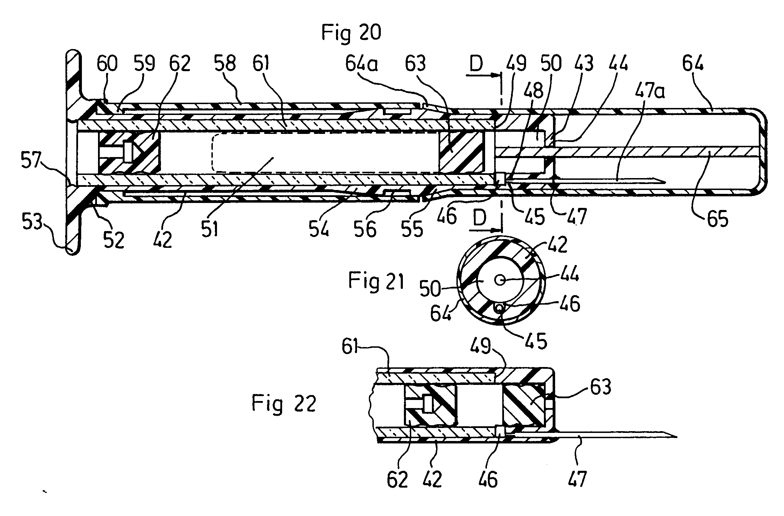

Le dispositif d'injection représenté aux figures 20 à 22 est quant à lui plus spécifiquement conçu pour pouvoir injecter de faibles doses de liquide.

Ce dispositif d'injection comprend un corps de seringue tubulaire 42 obturé, au droit de son extrémité antérieure par une paroi frontale 43 percée d'un orifice 44.

Ce corps de seringue comporte, en outre, un alésage longitudinal 45 ménagé dans sa paroi périphérique et s'étendant entre la paroi frontale 43 et un conduit radial interne 46, débouchant à l'intérieur dudit corps de seringue et ménagé à distance de ladite paroi frontale.

Cet alésage longitudinal 45 loge une aiguille d'injection 47 et comporte un épaulement interne intermédiaire 48 de butée de l'extrémité postérieure de ladite aiguille d'injection, adapté pour que cette dernière présente un tronçon actif d'injection 47a dans le prolongement de la paroi frontale 43.

Le corps de seringue comprend, en outre, un épaulement interne 49 juxtaposé longitudinalement au conduit radial 46, adapté pour délimiter à l'intérieur dudit corps de seringue, une chambre d'extrémité 50 incorporant ledit conduit radial, de moindre section que la section interne courante de ce corps de seringue.

Par ailleurs, ce corps de seringue 42 comporte deux lumières longitudinales telles que 51, un épaulement postérieur externe 52 de part et d'autre duquel s'étendent deux ailettes de prise digitale telles que 53, et un bossage formé de deux rampes longitudinales 54, 55 s'étendant de part et d'autre d'une rainure annulaire 56.

Enfin, ce corps de seringue 42 comporte, au droit de l'extrémité postérieure de l'épaulement 52, une nervure annulaire interne 57 de blocage en translation du récipient rigide pré-rempli.

Le fourreau 58 de ce dispositif d'injection est quant à lui strictement identique à ceux décrits précédemment et comprend donc une nervure interne postérieure 59 agencée pour coulisser le long du corps de seringue 42, et un épaulement postérieur 60 de butée contre l'épaulement 52 dudit corps de seringue.

Par ailleurs, le récipient rigide est constitué d'une cartouche 61 se présentant sous la forme d'un tube cylindrique de dimensions adaptées pour venir se loger dans le corps de seringue 42 et s'étendre entre l'épaulement interne 49 et la nervure annulaire postérieure 57 de ce dernier.

Cette cartouche 61 est obturée, au niveau de son extrémité postérieure, par un bouchon 62 faisant office de piston, associé à une tige de piston (non représentée) et au niveau de son extrémité antérieure, par un deuxième bouchon 63 adapté, tel que représenté à la figure 22, pour venir se loger dans la chambre d'extrémité 50 du corps de seringue dans une position où il vient en butée contre la paroi frontale 43 et dégage l'accès au conduit radial 46, lors du coulissement du bouchon postérieur 62.

En dernier lieu, ce dispositif d'injection comporte un capuchon 64 apte à venir coiffer le tronçon antérieur du corps de seringue 42 et à loger le tronçon actif 47a de l'aiguille d'injection 47.

Ce capuchon 64 de forme générale cylindrique présente un tronçon d'extrémité postérieur 64a évasé de forme conjuguée de la rampe d'accès 55 du corps de seringue 42, apte à venir coopérer avec cette dernière.

De plus, une tige axiale 65 logée à l'intérieur de ce capuchon 64 est adaptée pour s'étendre au travers de l'orifice 44 de la paroi frontale 42 du corps de seringue 42, de façon à venir au contact du bouchon 63 de la cartouche 61. Ainsi, tout déplacement de ce bouchon 63 et donc tout écoulement accidentel du liquide sont interdits tant que le capuchon 64 est en place.

Les principes de montage et d'utilisation de ce dispositif d'injection sont strictement identiques à ceux des dispositifs d'injection décrits ci-dessus.

De plus, de par sa conception, ce dispositif d'injection présente un volume "mort" très réduit et se trouve particulièrement adapté pour l'injection de faibles doses de liquides. En outre, du fait de la position de l'aiguille d'injection 47 disposée latéralement par rapport au corps de seringue 42, et moyennant une orientation correcte du biseau du tronçon actif d'injection 47a de cette dernière, ce dispositif d'injection est particulièrement adapté pour permettre une injection (par exemple dans le ventre) nécessitant de maintenir l'aiguille d'injection dans une position inclinée par rapport au point d'injection.

## Revendications

1. "Procédé de fabrication d'un dispositif d'injection pré-rempli comportant une aiguille d'injection (116; 15; 23; 47) qui consiste:
- à réaliser un corps de seringue tubulaire (101; 121; 1; 20; 42) en un matériau plastique doté vers une de ses extrémités, dite postérieure, d'organes (105; 5; 26; 53) de prise digitale, et vers son autre extrémité, dite antérieure, d'un organe externe de blocage (104; 123; 7-9; 27-29; 55-56),
- à réaliser un fourreau tubulaire (109; 10; 31; 58) de forme adaptée pour pouvoir coulisser le long du corps de seringue (101; 121; 1; 20; 42), comportant un organe interne de blocage (110; 11; 11; 32; 59) conjugué de celui dudit corps de seringue, ménagé vers une de ses extrémités, dite postérieure,
- à introduire dans le corps de seringue (101; 121; 1; 20; 42) un récipient rigide pré-rempli (114; 13; 34; 61) de section externe conjuguée de la section interne dudit corps de seringue, ledit récipient rigide étant doté de moyens (119; 120; 18; 19; 37; 38; 62) d'injection du liquide contenu dans ce dernier,
caractérisé en ce que:
- les organes (105; 5; 26; 53) de prise digitale et le corps de seringue forment une seule piéce,
- le corps de la seringue est en matériau déformable et l'organe externe de blocage (104; 123; 7-9; 27-29; 55-56) se déforme par déformation radiale entre une position rétractée et une position dilatée,
- le procédé comporte l'operation de présenter l'extrémité postérieure du fourreau (109; 10; 31; 58) en regard de l'extrémité antérieure du corps de seringue (101; 121; 1; 20; 42), et de faire coulisser relativement lesdits fourreau (109; 10; 31; 58) et corps de seringue, ce coulissement étant autorisé grâce à la rétraction radiale de l'organe de blocage dudit corps de seringue, jusqu'à amener l'extrémité postérieure du fourreau (109; 10; 31; 58) en butée à proximité des organes (105; 5; 26; 53) de prise digitale du corps de seringue (101; 121; 1; 20; 42), dans une position, dite d'injection, où ledit fourreau (109; 10; 31; 58) s'étend extérieurement le long du corps de seringue (101; 121; 1; 20; 42), et où au moins l'extrémité avant dudit corps de seringue (101; 121; 1; 20; 42) est dégagée,
- le récipient rigide pré-rempli est adapté pour amener l'organe externe de blocage (104; 123; 7-9; 27-29; 54-56) du corps de seringue dans sa position dilatée et interdire toute déformation radiale ultérieure dudit organe de blocage, et
- le procédé comprend l'opération de bloquer relativement en translation le corps de seringue (101; 121; 1; 20; 42) et le récipient rigide (114; 13; 34; 61).

2. Procédé selon la revendication 1, caractérisé en ce que l'on réalise un corps de seringue (101 ; 1 ; 20 ; 42) comportant une paroi périphérique dotée d'au moins une fente longitudinale (102 ; 2 ; 24 ; 51) au droit de l'organe externe de blocage (104 ; 7-9 ; 27-29 ; 54-56), apte à permettre les déformations radiales dudit organe de blocage.

3. Procédé selon la revendication 1, caractérisé en ce que l'on réalise un corps de seringue (121) comportant un tronçon d'extrémité antérieure (122) de forme tronconique de section décroissante en direction de l'extrémité antérieure dudit corps de seringue, et sur lequel est disposé l'organe externe de blocage (123), ledit tronçon tronconique étant doté longitudinalement de zones de faiblesse (124) aptes à permettre sa dilatation radiale.

4. Procédé selon l'une des revendications précédentes, caractérisé en ce que l'on solidarise les extrémités postérieures respectives du fourreau (109 ; 10 ; 31 ; 58) et du corps de seringue (101 ; 121 ; 1 ; 20 ; 42), dans la position d'injection de ces derniers, par des moyens de maintien aptes à pouvoir céder sous l'effet d'un effort de traction longitudinale exercée sur le fourreau et visant à le faire coulisser vers l'avant relativement au corps de seringue.

5. Procédé selon l'une des revendications précédentes, caractérisé en ce que :
- on réalise un corps de seringue (101 ; 1) doté d'une extrémité antérieure présentant la forme d'un manchon (3),
- on introduit dans le corps de seringue (101 ; 1) un récipient rigide constitué d'une seringue pré-remplie (114 ; 13) portant une aiguille d'injection (116 ; 15), et présentant une portion antérieure de forme adaptée pour venir se loger dans le manchon (3) du corps de seringue (101 ; 1), de façon que l'aiguille d'injection (116 ; 15) s'étende dans le prolongement dudit corps de seringue.

6. Procédé selon l'une des revendications 1 ou 4, caractérisé en ce que :
- on réalise un corps de seringue (20) doté, vers son extrémité antérieure, d'un nez (21) percé longitudinalement d'un alésage (22) de section adaptée pour loger une aiguille d'injection (23),
- on introduit une aiguille d'injection (23) dans l'alésage (22) du nez (21) du corps de seringue (20), de façon que cette dernière présente un tronçon actif d'injection (23a) dans le prolongement dudit corps de seringue, et un tronçon de percement (23b) débouchant à l'intérieur de celui-ci,
- on solidarise l'aiguille d'injection (23) dans l'alésage (22) du nez (21) du corps de seringue (20),
- et on introduit dans le corps de seringue (20) un récipient rigide pré-rempli (34) doté d'un organe d'obturation (35) apte à être percé par le tronçon de percement (23b) de l'aiguille d'injection (23).

7. Procédé selon l'une des revendications 1 ou 4, caractérisé en ce que :
- on réalise un corps de seringue (42) doté d'une extrémité antérieure obturée par une paroi d'extrémité frontale (43) percée d'un orifice (44), et comportant un alésage longitudinal (45) ménagé dans sa paroi périphérique, s'étendant entre ladite paroi frontale et un conduit radial interne (46) débouchant à l'intérieur dudit corps de seringue, ce conduit radial (46) étant ménagé à distance de la paroi frontale (43) et étant juxtaposé longitudinalement à un épaulement (49) délimitant, à l'intérieur du corps de seringue (42), une chambre d'extrémité (50), incorporant ledit conduit radial, de moindre section que la section interne courante dudit corps de seringue,
- on introduit une aiguille d'injection (47) dans l'alésage longitudinal (45) de façon que cette dernière possède un tronçon actif d'injection (47a) dans le prolongement de la paroi frontale (43) du corps de seringue (42),
- on solidarise l'aiguille d'injection (47) dans l'alésage longitudinal (45) du corps de seringue (42),
- et on introduit, à l'intérieur du corps de seringue (42), un tube rigide pré-rempli (61) de section adaptée pour venir en butée contre l'épaulement interne (49) dudit corps de seringue, ledit tube rigide étant obturé, vers chacune de ses extrémités, par un bouchon coulissant (62, 63), dit respectivement antérieur et postérieur, le bouchon antérieur (63) présentant une forme adaptée pour venir se loger dans la chambre d'extrémité (50) du corps de seringue (42), lors du coulissement du bouchon postérieur (62), dans une position où il vient buter contre la paroi frontale (43) et dégage le conduit radial (46).

8. Procédé selon l'une des revendications précédentes, caractérisé en ce que l'on encapuchonne l'extrémité avant du corps de seringue (101 ; 121 ;1 ; 20 ; 42).

9. Dispositif d'injection du type pré-rempli comportant une aiguille d'injection (116; 15; 23; 47), qui comprend en combinaison:
- un corps de seringue tubulaire (101; 121; 1; 20; 42) réalisé en un matériau plastique, doté vers une de ses extrémités, dite postérieure, d'organes (105; 5; 26; 53) de prise digitale, et vers son autre extrémité, dite antérieure, d'un organe externe de blocage (104; 123; 7-9; 27-29; 54-56),
- un fourreau tubulaire (109; 10; 31; 58) de section interne adaptée pour pouvoir coulisser le long du corps de seringue (101; 121; 1; 20; 42), doté d'un organe interne de blocage (110; 111; 11; 32; 59) conjugué de celui dudit corps de seringue, ménagé vers une de ses extrémités dite postérieure, ledit fourreau étant apte à pouvoir coulisser entre deux positions:
- une position arrière, d' injection, dans laquelle, d'une part, l'extrémité postérieure du fourreau (109; 10; 31; 58) se trouve en butée à proximité des organes (105; 5; 26; 53) de prise digitale du corps de seringue (101; 121; 1; 20; 42), et d'autre part, au moins l'extrémité avant du corps de seringue (101; 121; 1; 20; 42) est dégagée,
- une position avant, de protection après usage, dans laquelle, d'une part les organes de blocage respectifs (104; 110; 123 110; 111; 7-9; 11; 27-29; 32; 54-56; 59) du fourreau (109; 10; 31; 58) et du corps de seringue (101; 121; 1; 20; 42) sont en état de coopération, et d'autre part, le fourreau recouvre l'extrémité avant du corps de seringue et s'étend dans le prolongement de ce dernier,
- un récipient rigide pré-rempli (114; 13; 34; 61) introduit à l'intérieur du corps de seringue (101; 121; 1; 20; 42) et de section externe conjuguée de la section interne dudit corps de seringue,
caractérisé en ce que:
- les organes (105; 5; 26; 53) de prise digitale et le corps de seringue forment une seule piéce,
- le corps de la seringue est en matériau déformable et l'organe externe de blocage (104; 123; 7-9; 27-29; 55-56) se déforme par déformation radiale entre une position rétractée et une position dilatée, et
- le dispositif comprend des moyens de blocage (106; 6; 30; 57) relatif en translation du récipient rigide (114; 13; 34; 61) et du corps de seringue (101; 121; 1; 20; 42).

10. Dispositif d'injection selon la revendication 9, caractérisé en ce que les extrémités postérieures respectives du fourreau (109 ; 10 ; 31 ; 58) et du corps de seringue (101 ; 121 ; 1 ; 20 ; 42) sont solidarisées, dans la position arrière d'injection de ces derniers, par des moyens de maintien aptes à pouvoir céder sous l'effet d'un effort de traction longitudinale exercée sur le fourreau visant à le faire coulisser vers sa position avant de protection après usage.

11. Dispositif d'injection selon l'une des revendications 9 ou 10, caractérisé en ce que le corps de seringue (121) comporte un tronçon d'extrémité antérieure (122) de section décroissante en direction de l'extrémité antérieure dudit corps de seringue, et sur lequel est disposé l'organe externe de blocage (123), ledit tronçon tronconique étant doté longitudinalement de zones de faiblesse (124) aptes à permettre sa dilatation radiale.

12. Dispositif d'injection selon l'une des revendications 9 ou 10, caractérisé en ce que le corps de seringue (101 ; 1 ; 20 ; 42) comporte une paroi périphérique dotée d'au moins une fente longitudinale (102 ; 2 ; 24 ; 51 ) s'étendant au droit de l'organe externe de blocage (104 ; 123 ; 7-9 ; 27-29 ; 54-56), apte à permettre les déformations radiales dudit organe externe de blocage.

13. Dispositif d'injection selon la revendication 12, caractérisé en ce que le corps de seringue (101 ; 1 ; 20 ; 42) comporte une paroi périphérique dotée de deux fentes longitudinales (102 ; 2 ; 24 ; 51) diamétralement opposées.

14. Dispositif d'injection selon la revendication 13, caractérisé en ce que le corps de seringue (101) présente un diamètre interne sensiblement supérieur à celui du diamètre externe du récipient rigide (114), et comporte des nervures internes longitudinales (103) au droit de l'organe interne de blocage (104) dudit corps de seringue, déterminant un diamètre conjugué du diamètre externe du récipient rigide (114).

15. Dispositif d'injection selon l'une des revendications 9 à 14, caractérisé en ce que :
- l'organe de blocage ménagé sur un des éléments, corps de seringue ou fourreau présente la forme d'un bossage annulaire comportant une rainure annulaire (7 ; 29 ; 56) et des crans (110, 111 ; 9 ; 27, 28 ; 54, 55), disposés de part et d'autre de cette dernière,
- l'organe de blocage ménagé sur l'autre élément, fourreau ou corps de seringue, est constitué d'une nervure annulaire (104 ; 123 ; 11 ; 32 ; 59) délimitant une section conjuguée de la section externe du fond de la rainure du bossage du premier élément.

16. Dispositif d'injection selon la revendication 15, caractérisé en ce que les crans (110) de l'organe de blocage de l'un des éléments (109), corps de seringue ou fourreau, situés vers l'extrémité dudit élément présentent une épaisseur supérieure à celle des deuxièmes crans (111) délimitant la rainure dudit organe de blocage.

17. Dispositif d'injection selon la revendication 16, caractérisé en ce que les deuxièmes crans (111) de l'organe de blocage de l'élément (109), corps de seringue ou fourreau, présentent une face frontale s'étendant selon une corde dudit élément, adaptée pour obtenir au niveau desdits crans un périmètre au moins égal à celui de l'autre élément (101) au droit de la nervure annulaire (104) de ce dernier.

18. Dispositif d'injection selon l'une des revendications 9 à 17, caractérisé en ce que :
- le corps de seringue (101 ; 1) comporte une extrémité antérieure présentant la forme d'un manchon (3),
- le récipient rigide pré-rempli est constitué d'une seringue pré-remplie (114 ; 13) portant une aiguille d'injection (116 ; 15), présentant une portion antérieure de forme adaptée pour venir se loger dans le manchon (3) du corps de seringue (101 ; 1), de façon que l'aiguille d'injection (116 ; 15) s'étende dans le prolongement dudit corps de seringue.

19. Dispositif d'injection selon la revendication 18 dans lequel la seringue pré-remplie (114 ; 13) comporte une extrémité postérieure dotée d'une collerette (117 ; 16) de prise digitale, caractérisé en ce que le corps de seringue (101 ; 1) comprend, au droit de son extrémité postérieure, des crans radiaux (106 ; 6) aptes à loger la collerette (117 ; 16) et à assurer le blocage en translation de la seringue pré-remplie (114 ; 13).

20. Dispositif d'injection selon la revendication 19, caractérisé en ce que les crans radiaux (106) sont ménagés dans une bague (105) en forme de coupelle de diamètre supérieur à celui du corps de seringue (101), disposée dans le prolongement postérieur dudit corps de seringue, ladite bague étant adaptée pour servir de butée frontale de l'extrémité postérieure du fourreau (109).

21. Dispositif d'injection selon la revendication 20, caractérisé en ce qu'il comprend un jonc annulaire déformable (107) agencé pour être sollicité par la collerette (117) de la seringue (114), de façon à assurer un blocage sans jeu de cette dernière dans les crans radiaux (106).

22. Dispositif d'injection selon l'une des revendications 18 à 21 dans lequel la seringue pré-remplie (114 ; 13) est obturée par un capuchon amovible (118 ; 17) logeant l'aiguille d'injection (116 ; 15), avant injection.

23. Dispositif d'injection selon l'une des revendications 9 à 17, caractérisé en ce que :
- le corps de seringue (20) est doté, vers son extrémité antérieure, d'un nez (21) percé longitudinalement d'un alésage (22) logeant une aiguille d'injection (23) de façon que cette dernière présente un tronçon actif d'injection (23a) dans le prolongement dudit corps de seringue, et un tronçon de percement (23b) débouchant à l'intérieur de celui-ci,
- le récipient rigide pré-rempli est constitué d'une cartouche (34) dotée d'un organe d'obturation (35) apte à être percé par le tronçon de percement (23b) de l'aiguille d'injection (23).

24. Dispositif d'injection selon la revendication 23, caractérisé en ce que l'organe d'obturation de la cartouche (34) consiste en un bouchon (35) apte à être percé, disposé, dans une phase initiale, de façon à se trouver à distance de l'extrémité du tronçon de percement (23b) de l'aiguille d'injection (23), ladite cartouche étant obturée vers son autre extrémité, par un deuxième bouchon (37) faisant office de piston.

25. Dispositif d'injection selon l'une des revendications 23 ou 24, caractérisé en ce que le corps de seringue (20) comprend, au droit de son extrémité postérieure, une nervure interne (30) apte à assurer le blocage en translation de la cartouche (34) introduite dans ledit corps de seringue.

26. Dispositif d'injection selon l'une des revendications 23 à 25, caractérisé en ce qu'il comprend un capuchon (39) apte à loger le tronçon actif (23a) de l'aiguille d'injection (23), ledit capuchon et le nez (21) du corps de seringue (20) étant dotés d'organes d'encliquetage conjugués (40, 41) aptes à permettre de les solidariser de façon amovible.

27. Dispositif d'injection selon l'une des revendications 9 à 17, caractérisé en ce que :
- le corps de seringue (42) est doté d'une extrémité antérieure obturée par une paroi d'extrémité frontale (43) percée d'un orifice (44), et comporte un alésage longitudinal (45) ménagé dans sa paroi périphérique logeant une aiguille d'injection (47) de façon que cette dernière présente un tronçon actif d'injection (47a) dans le prolongement de la paroi frontale (43), ledit alésage s'étendant entre ladite paroi frontale et un conduit radial interne (46) débouchant à l'intérieur dudit corps de seringue, ce conduit radial (46) étant ménagé à distance de la paroi frontale (43) et étant juxtaposé longitudinalement à un épaulement (49) délimitant, à l'intérieur du corps de seringue (42), une chambre d'extrémité (50) incorporant ledit conduit radial, de moindre section que la section interne courante dudit corps de seringue,
- le récipient rigide pré-rempli est constitué d'une cartouche (61) de section adaptée pour venir en butée contre l'épaulement interne (49) du corps de seringue (42), ladite cartouche étant obturée, vers chacune de ses extrémités, par un bouchon coulissant (62, 63), dit respectivement antérieur et postérieur, le bouchon postérieur (62) faisant office de piston, et le bouchon antérieur (63) présentant une forme adaptée pour venir se loger dans la chambre d'extrémité (50) du corps de seringue (42), lors de l'actionnement du bouchon postérieur (62), dans une position où il vient en butée contre la paroi frontale (43) et dégage l'accès au conduit radial (46).

28. Dispositif d'injection selon la revendication 27, caractérisé en ce que l'alésage longitudinal (45) présente un épaulement interne intermédiaire (48) de butée de l'extrémité postérieure de l'aiguille d'injection (47).

29. Dispositif d'injection selon l'une des revendications 27 ou 28, caractérisé en ce que le corps de seringue (42) comprend, au droit de son extrémité postérieure, une nervure interne (57) apte à assurer le blocage en translation de la cartouche (61) introduite dans ledit corps de seringue.

30. Dispositif d'injection selon l'une des revendications 27 à 29, caractérisé en ce qu'il comporte un capuchon (64) apte à venir coiffer le tronçon antérieur du corps de seringue (42) et à loger le tronçon actif (47a) de l'aiguille d'injection (47).

## Claims

1. A process for manufacturing a pre-filled injection device comprising an injection needle (116; 15; 23; 47), which comprises:
- producing a tubular syringe body (101; 121; 1; 20; 42) of a plastics material equipped towards one of its ends, called the rear end, with finger-hold elements (105; 5; 26; 53) and towards its other end, called the front end, with an external locking element (104; 123; 7-9; 27-29; 55-56),
- producing a tubular sheath (109; 10; 31; 58) of a shape suitable for being able to slide along the syringe body (101; 121; 1; 20; 42), comprising an internal locking element (110, 111; 11; 32; 59) paired with that of the said syringe body, arranged towards one of its ends, called the rear end,
- introducing into the syringe body (101; 121; 1; 20; 42) a rigid pre-filled container (114; 13; 34; 61) of external cross-section paired with the internal cross-section of the said syringe body, the said rigid container being equipped with means (119; 120; 18; 19; 37; 38; 62) for injecting the liquid contained in the latter,
characterised in that:
- the finger-hold elements (105; 5; 26; 53) and the syringe body form a single part,
- the syringe body is of deformable material and the external locking element (104; 123; 7-9; 27-29; 55-56) deforms by radial deformation between a retracted position and a dilated position,
- the process comprises the operation of offering up the rear end of the sheath (109; 10; 31; 58) opposite the front end of the syringe body (101; 121; 1; 20; 42) and causing the said sheath (109; 10; 31; 58) and syringe body to slide with respect to each other, this sliding being permitted thanks to the radial retraction of the locking element of the said syringe body, so as to bring the rear end of the sheath (109; 10; 31; 58) to rest adjacent to the finger-hold elements (105; 5; 26; 53) of the syringe body (101; 121; 1; 20; 42) in a position, called the injection position, in which the said sheath (109; 10; 31; 58) extends externally along the syringe body (101; 121; 1; 20; 42) and in which at least the front end of the said syringe body (101; 121; 1; 20; 42) is disengaged,
- the rigid pre-filled container is suitable for bringing the external locking element (104; 123; 7-9; 27-29; 54-56) of the syringe body into its dilated position and preventing any further radial deformation of the said locking element, and
- the process comprises the operation of locking the syringe body (101; 121; 1; 20; 42) and the rigid container (114; 13; 34; 61) in translation with respect to each other.

2. A process according to claim 1, characterised in that a syringe body (101; 1; 20; 42) is produced comprising a peripheral wall equipped with at least one longitudinal slot (102; 2; 24; 51) at the level of the external locking element (104; 7-9; 27-29; 54-56), suitable for permitting the radial deformations of the said locking element.

3. A process according to claim 1, characterised in that a syringe body (121) is produced comprising a front end portion (122) in the shape of a truncated cone of decreasing cross-section in the direction of the front end of the said syringe body, and on which the external locking element (123) is arranged, the said truncated-cone-shaped portion being equipped longitudinally with weakness zones (124) suitable for permitting its radial dilation.

4. A process according to one of the preceding claims, characterised in that the respective rear ends of the sheath (109; 10; 31; 58) and of the syringe body (101; 121; 1; 20; 42) are secured, in their injection position, by holding means suitable for being able to yield under the effect of a longitudinal tensile force applied to the sheath and intended to make it slide towards the front with respect to the syringe body.

5. A process according to one of the preceding claims, characterised in that:
- a syringe body (101; 1) is produced which is equipped with a front end having the form of a sleeve (3),
- a rigid container comprising a pre-filled syringe (114; 13) with an injection needle (116; 15) and having a front portion of a shape suitable for being accommodated in the sleeve (3) of the syringe body (101; 1), so that the injection needle (116; 15) extends in the extension of the said syringe body, is introduced into the syringe body (101; 1).

6. A process according to one of claims 1 or 4, characterised in that:
- a syringe body (20) is produced which is equipped towards its front end with a nose (21) pierced longitudinally by a bore (22) of cross-section suitable for accommodating an injection needle (23),
- an injection needle (23) is introduced into the bore (22) of the nose (21) of the syringe body (20), so that it has an active injection portion (23a) in the extension of the said syringe body, and a piercing portion (23b) opening into the interior thereof,
- the injection needle (23) is secured in the bore (22) of the nose (21) of the syringe body (20),
- and a rigid, pre-filled container (34) equipped with a sealing element (35) suitable for being pierced by the piercing portion (23b) of the injection needle (23) is introduced into the syringe body (20).

7. A process according to one of claims 1 or 4, characterised in that:
- a syringe body (42) is produced which is equipped with a front end sealed by a front end wall (43) pierced by an aperture (44), and comprises a longitudinal bore (45) arranged in its peripheral wall, extending between the said front wall and a radial internal duct (46) opening in the interior of the said syringe body, this radial duct (46) being arranged at a distance from the front wall (43) and being juxtaposed longitudinally to a shoulder (49) delimiting, inside the syringe body (42), an end chamber (50), incorporating the said radial duct, of smaller cross-section than the standard internal cross-section of the said syringe body,
- an injection needle (47) is introduced into the longitudinal bore (45) so that it has an active injection portion (47a) in the extension of the front wall (43) of the syringe body (42),
- the injection needle (47) is secured in the longitudinal bore (45) of the syringe body (42),
- and into the interior of the syringe body (42) is introduced a rigid pre-filled tube (61) of cross-section suitable for coming to rest against the internal shoulder (49) of the said syringe body, the said rigid tube being sealed, towards each of its ends, by a sliding plug (62, 63), respectively called front and rear, the front plug (63) having a shape suitable for being accommodated in the end chamber (50) of the syringe body (42), when the rear plug (62) slides, in a position in which it comes to rest against the front wall (43) and disengages the radial duct (46).

8. A process according to one of the preceding claims, characterised in that the front end of the syringe body (101; 121; 1; 20; 42) is capped.

9. An injection device of the pre-filled type comprising an injection needle (116; 15; 23; 47) which comprises in combination:
- a tubular syringe body (101; 121; 1; 20; 42) made of a plastics material, equipped towards one of its ends, called the rear end, with finger-hold elements (105; 5; 26; 53) and towards its other end, called the front end, with an external locking element (104; 123; 7-9; 27-29; 54-56),
- a tubular sheath (109; 10; 31; 58) of internal cross-section suitable for being able to slide along the syringe body (101; 121; 1; 20; 42), equipped with an internal locking element (110, 111; 11; 32; 59) paired with that of the said syringe body, arranged towards one of its ends, called the rear end, the said sheath being suitable for being able to slide between two positions:
- a rear, injection, position in which the rear end of the sheath (109; 10; 31; 58) rests adjacent to the finger-hold elements (105; 5; 26; 53) of the syringe body (101; 121; 1; 20; 42) on the one hand, and at least the front end of the syringe body (101; 121; 1; 20; 42) is disengaged on the other hand,
- a front, post-use protection, position in which the respective locking elements (104; 110, 123, 110, 111; 7-9; 11; 27-29; 32; 54-56; 59) of the sheath (109; 10; 31; 58) and of the syringe body (101; 121; 1; 20; 42) are in a state of cooperation on the one hand, and the sheath covers the front end of the syringe body and extends in the extension thereof on the other hand,
- a rigid pre-filled container (114; 13; 34; 61) introduced into the interior of the syringe body (101; 121; 1; 20; 42) and of external cross-section paired with the internal cross-section of the said syringe body,
characterised in that:
- the finger-hold elements (105; 5; 26; 53) and the syringe body form a single part,
- the syringe body is of deformable material and the external locking element (104; 123; 7-9; 27-29; 55-56) deforms by radial deformation between a retracted position and a dilated position, and
- the device comprises means (106; 6; 30; 57) for the relative locking in translation of the rigid container (114; 13; 34; 61) and the syringe body (101; 121; 1; 20; 42).

10. An injection device according to claim 9, characterised in that the respective rear ends of the sheath (109; 10; 31; 58) and of the syringe body (101; 121; 1; 20; 42) are secured, in their rear injection position, by holding means suitable for being able to yield under the effect of a longitudinal tensile force applied to the sheath and intended to make it slide towards its front, post-use protection, position.

11. An injection device according to one of claims 9 or 10, characterised in that the syringe body (121) comprises a front end portion (122) of decreasing cross-section in the direction of the front end of the said syringe body, on which portion the external locking element (123) is arranged, the said truncated-cone-shaped portion being equipped longitudinally with weakness zones (124) suitable for permitting its radial dilation.

12. An injection device according to one of claims 9 or 10, characterised in that the syringe body (101; 1; 20; 42) comprises a peripheral wall equipped with at least one longitudinal slot (102; 2; 24; 51) extending at the level of the external locking element (104; 123; 7-9; 27-29; 54-56), suitable for permitting the radial deformations of the said external locking element.

13. An injection device according to claim 12, characterised in that the syringe body (101; 1; 20; 42) comprises a peripheral wall equipped with two diametrically opposed longitudinal slots (102; 2; 24; 51).

14. An injection device according to claim 13, characterised in that the syringe body (101) has an internal diameter appreciably larger than the external diameter of the rigid container (114) and comprises longitudinal internal ribs (103) at the level of the internal locking element (104) of the said syringe body, determining a diameter paired with the external diameter of the rigid container (114).

15. An injection device according to one of claims 9 to 14, characterised in that:
- the locking element arranged on one of the components, syringe body or sheath, has the form of an annular boss comprising an annular groove (7; 29; 56) and notches (110, 111; 9; 27, 28; 54, 55), arranged either side thereof,
- the locking element arranged on the other component, sheath or syringe body, comprises an annular rib (104; 123; 11; 32; 59) delimiting a section paired with the external section of the bottom of the groove of the boss of the first component.

16. An injection device according to claim 15, characterised in that the notches (110) of the locking element of one of the components (109), syringe body or sheath, located towards the end of the said component, have a thickness greater than that of the second notches (111) delimiting the groove of the said locking element.

17. An injection device according to claim 16, characterised in that the second notches (111) of the locking element of the component (109), syringe body or sheath, have a front face which extends according to a chord of the said component and is suitable for obtaining at the said notches a perimeter at least equal to that of the other component (101) at the level of the latter's annular rib (104).

18. An injection device according to one of claims 9 to 17, characterised in that:
- the syringe body (101; 1) comprises a front end having the form of a sleeve (3),
- the pre-filled rigid container comprises a pre-filled syringe (114; 13) having an injection needle (116; 15), having a front portion of a shape suitable for being accommodated in the sleeve (3) of the syringe body (101; 1), so that the injection needle (116; 15) extends in the extension of the said syringe body.

19. An injection device according to claim 18 in which the pre-filled syringe (114; 13) comprises a rear end equipped with a finger-hold flange (117; 16), characterised in that the syringe body (101; 1) comprises, at its rear end, radial notches (106; 6) suitable for accommodating the flange (117; 16) and for ensuring the locking in translation of the pre-filled syringe (114; 13).

20. An injection device according to claim 19, characterised in that the radial notches (106) are arranged in a cup-shaped ring (105) of a diameter greater than that of the syringe body (101) and arranged in the rear extension of the said syringe body, the said ring being suitable for serving as a frontal rest for the rear end of the sheath (109).

21. An injection device according to claim 20, characterised in that it comprises an annular deformable band (107) arranged to be acted on by the flange (117) of the syringe (114), so as to ensure a play-free locking thereof in the radial notches (106).

22. An injection device according to one of claims 18 to 21, in which the pre-filled syringe (114; 13) is sealed by a detachable cap (118; 17) housing the injection needle (116; 15), before injection.

23. An injection device according to one of claims 9 to 17, characterised in that:
- the syringe body (20) is equipped, towards its front end, with a nose (21) pierced longitudinally by a bore (22) accommodating an injection needle (23) so that the latter has an active injection portion (23a) in the extension of the said syringe body, and a piercing portion (23b) opening into the interior thereof,
- the rigid, pre-filled container comprises a cartridge (34) equipped with a sealing element (35) suitable for being pierced by the piercing portion (23b) of the injection needle (23).

24. An injection device according to claim 23, characterised in that the sealing element of the cartridge (34) comprises a plug (35) suitable for being pierced and arranged, in an initial phase, so as to be at a distance from the end of the piercing portion (23b) of the injection needle (23), the said cartridge being sealed towards its other end, by a second plug (37) performing the function of plunger.

25. An injection device according to one of claims 23 or 24, characterised in that the syringe body (20) comprises, at its rear end, an internal rib (30) suitable for ensuring the locking in translation of the cartridge (34) introduced into the said syringe body.

26. An injection device according to one of claims 23 to 25, characterised in that it comprises a cap (39) suitable for housing the active portion (23a) of the injection needle (23), the said cap and the nose (21) of the syringe body (20) being equipped with paired latching elements (40, 41) suitable for enabling them to be secured in a detachable manner.

27. An injection device according to one of claims 9 to 17, characterised in that:
- the syringe body (42) is equipped with a front end sealed by a front end wall (43) pierced by an aperture (44), and comprises a longitudinal bore (45) arranged in its peripheral wall housing an injection needle (47) so that the latter has an active injection portion (47a) in the extension of the front wall (43), the said bore extending between the said front wall and a radial internal duct (46) opening in the interior of the said syringe body, this radial duct (46) being arranged at a distance from the front wall (43) and being juxtaposed longitudinally to a shoulder (49) delimiting, inside the syringe body (42), an end chamber (50) incorporating the said radial duct, of smaller cross-section than the standard internal cross-section of the said syringe body,
- the rigid pre-filled container comprises a cartridge (61) of cross-section suitable for coming to rest against the internal shoulder (49) of the syringe body (42), the said cartridge being sealed, towards each of its ends, by a sliding plug (62, 63), respectively called front and rear, the rear plug (62) performing the function of plunger, and the front plug (63) having a shape suitable for being accommodated in the end chamber (50) of the syringe body (42), when the rear plug (62) is actuated, in a position in which it comes to rest against the front wall (43) and disengages the access to the radial duct (46).

28. An injection device according to claim 27, characterised in that the longitudinal bore (45) has an internal intermediate shoulder (48) as a rest for the rear end of the injection needle (47).

29. An injection device according to one of claims 27 or 28, characterised in that the syringe body (42) comprises, at its rear end, an internal rib (57) suitable for ensuring the locking in translation of the cartridge (61) introduced into the said syringe body.

30. An injection device according to one of claims 27 to 29, characterised in that it comprises a cap (64) suitable for covering the front portion of the syringe body (42) and for housing the active portion (47a) of the injection needle (47).

## Patentansprüche

1. Verfahren zur Herstellung einer vorgefüllten Injektionsvorrichtung, umfassend eine Injektionsnadel (116; 15; 23; 47), umfassend die folgenden Schritte:
- Bereitstellen eines röhrenförmigen Spritzenkörpers (101; 121; 1; 20; 42) aus einem Plastikmaterial, das an einem seiner Enden, hinteres Ende genannt, mit Fingergriffteilen (105; 5; 26; 53) und an seinem anderen Ende, vorderes Ende genannt, mit einem äußeren Sperrteil (104; 123; 7-9; 27-29; 55-56) versehen ist,
- Bereitstellen einer röhrenförmigen Hülse (109; 10; 31; 58), die so ausgestaltet ist, daß sie an dem Spritzenkörper (101; 121; 1; 20; 42) entlang gleiten kann, und die ein inneres Sperrteil (110, 111;11; 32; 59) umfaßt, das mit dem des gennanten Spritzenkörpers verbunden ist, das an einem seiner Enden, hinteres Ende genannt, angeordnet ist,
- Einführen eines starren vorgefüllten Gefäßes (114; 13; 34; 61) in den Spritzenkörper (101; 121; 1; 20;42), das einen Außenquerschnitt hat, der zum Innenquerschnitt des genannten Spritzenkörpers paßt, wobei das genannte starre Gefäß mit Mitteln (119; 120; 18; 19; 37; 38; 62) zum Einspritzen der in Letzterem enthaltenen Flüssigkeit versehen ist,
dadurch gekennzeichnet, daß:
- die Fingergriffteile (105; 5; 26; 53) und der Spritzenkörper einstückig ausgebildet sind,
- der Spritzenkörper aus einem verformbaren Material besteht und das äußere Sperrteil (104; 123; 7-9; 27-29; 55-56) sich mit der radialen Verformung zwischen einer eingezogenen Position und einer ausgefahrenen Position verformt,
- das Verfahren den folgenden Vorgang umfaßt: Positionieren des hinteren Endes der Hülse (109; 10; 31; 58) gegenüber dem vorderen Ende des Spritzenkörpers (101; 121; 1; 20; 42) und Verschieben der genannten Hülse (109; 10; 31; 58) und des Spritzenkörpers relativ zueinander, wobei die Verschiebung durch ein radiales Einziehen des Sperrteils des genannten Spritzenkörpers bewirkt wird, bis das hintere Ende der Hülse (109; 10; 31; 58) an den Fingergriffteilen (105; 5; 26; 53) des Spritzenkörpers (101; 121; 1; 20; 42), in einer Position, Injektionsposition genannt, anschlägt, in der sich die genannte Hülse (109; 10; 31; 58) extern an dem Spritzenkörper (101; 121; 1; 20; 42) entlang erstreckt und in der wenigstens das Ende vor dem genannten Spritzenkörper (101; 121; 1; 20;42) freigegeben ist,
- das vorgefüllte starre Gefäß so ausgestaltet ist, daß es das äußere Sperrteil (104; 123; 7-9; 27-29; 54-56) des Spritzenkörpers in seine ausgefahrene Position führt und jegliche weitere radiale Verformung des genannten Sperrteils verhindert, und
- das Verfahren den Vorgang des Sperrens der relativen Verschiebung des Spritzenkörpers (101; 121; 1; 20; 42) und des starren Gefäßes (114; 13; 34; 61) gegeneinander umfaßt.

2. Verfahren nach Anspruch 1, dadurch gekennzeichnet, daß ein Spritzenkörper (101; 1; 20; 42) bereitgestellt wird, der eine Umfangswand aufweist, die wenigstens einen Längsschlitz (102; 2; 24; 51) in der Höhe des äußeren Sperrteils (104; 7-9; 27-29; 54-56) aufweist, der die radialen Verformungen des genannten Sperrteils ermöglicht.

3. Verfahren nach Anspruch 1, dadurch gekennzeichnet, daß ein Spritzenkörper (121) bereitgestellt wird, der ein Endstück des vorderen Endes (122) in kegelstumpfartiger Form mit einem Querschnitt aufweist, der in Richtung des vorderen Endes des genannten Spritzenkörpers abnimmt und an dem das äußere Sperrteil (123) angeordnet ist, wobei das genannte kegelstumpfförmige Endstück in Längsrichtung mit geschwächten Zonen (124) versehen ist, die das radiale Ausfahren ermöglichen.

4. Verfahren nach einem der vorherigen Ansprüche, dadurch gekennzeichnet, daß die jeweiligen hinteren Enden der Hülse (109; 10; 31; 58) und des Spritzenkörpers (101; 121; 1; 20; 42) in der Injektionsposition der Letzteren durch Haltemittel fixiert werden, die in der Lage sind, unter der Wirkung einer in Längsrichtung auf die Hülse ausgeübten Zugkraft nachzugeben, mit dem Ziel, sie relativ zum Spritzenkörper nach vorne zu verschieben.

5. Verfahren nach einem der vorherigen Ansprüche, dadurch gekennzeichnet, daß:
- ein Spritzenkörper (101; 1) bereitgestellt wird, der mit einem vorderen Ende in der Form einer Muffe (3) versehen ist,
- ein starres Gefäß in den Spritzenkörper (101; 1) eingeführt wird, das durch eine vorgefüllte Spritze (114; 13) gebildet wird, die eine Injektionsnadel (116; 15) trägt, und das einen vorderen Abschnitt aufweist, der so ausgestaltet ist, daß er auf eine solche Weise in der Muffe (3) des Spritzenkörpers (101; 1) zu liegen kommt, daß die Injektionsnadel (116; 15) in die Verlängerung des genannten Spritzenkörpers verläuft.

6. Verfahren nach einem der Ansprüche 1 und 4, dadurch gekennzeichnet, daß:
- ein Spritzenkörper (20) bereitgestellt wird, der an seinem vorderen Ende mit einer Nase (21) versehen ist, die in Längsrichtung mit einer Bohrung (22) versehen ist, die einen Querschnitt aufweist, der eine Injektionsnadel (23) aufnehmen kann,
- eine Injektionsnadel (23) in die Bohrung (22) der Nase (21) des Spritzenkörpers (20) eingeführt wird, so daß die Letztere ein aktives Injektionsendstück (23a) in der Verlängerung des genannten Spritzenkörpers und ein Durchbohrungsendstück (23b) aufweist, das ins Innere desselben vordringt,
- die Injektionsnadel (23) in der Bohrung (22) der Nase (21) des Spritzenkörpers (20) fixiert wird,
- ein starres vorgefülltes Gefäß (34) in den Spritzenkörper (20) eingeführt wird, der mit einem Verschlußteil (35) versehen ist, das von dem Durchbohrungsabschnitt (23b) der Injektionsnadel (23) durchbohrt werden kann.

7. Verfahren nach einem der Ansprüche 1 und 4, dadurch gekennzeichnet, daß:
- ein Spritzenkörper (42) bereitgestellt wird, der ein vorderes Ende aufweist, das von einer vorderen Endwand (43) verschlossen wird, die eine Bohrung (44) aufweist, und der eine Längsbohrung (45) aufweist, die in seiner Umfangswand vorgesehen ist und die zwischen der genannten Frontwand und einem radialen Innenkanal (46) verläuft, der ins Innere des genannten Spritzenkörpers führt, wobei dieser radiale Kanal (46) in einem Abstand von der Frontwand (43) vorgesehen ist und in Längsrichtung neben einem Ansatz (49) liegt, der im Inneren des Spritzenkörpers (42) eine Endkammer (50) begrenzt, die den genannten radialen Kanal aufnimmt, der einen geringeren Querschnitt aufweist als der laufende Innenquerschnitt des genannten Spritzenkörpers,
- eine Injektionsspritze (47) auf eine solche Weise in die Längsbohrung (45) eingeführt wird, daß Letztere ein aktives Injektionsendstück (47a) in der Verlängerung der Frontwand (43) des Spritzenkörpers (42) aufweist,
- die Injektionsnadel (47) in der Längsbohrung (45) des Spritzenkörpers (42) fixiert wird,
- eine starre vorgefüllte Röhre (61) in das Innere des Spritzenkörpers (42) eingeführt wird, die einen solchen Querschnitt aufweist, daß sie am inneren Ansatz (49) des genannten Spritzenkörpers anliegen kann, wobei die genannte starre Röhre an jedem ihrer Enden durch einen Gleitstopfen (62, 63) verschlossen ist, jeweils als vorderer Stopfen und hinterer stopfen bezeichnet, wobei der vordere Stopfen (63) eine solche Form aufweist, daß er in der Kammer des Endes (50) des Spritzenkörpers (42) während der Verschiebung des hinteren Stopfens (62) in einer Position aufgenommen werden kann, wo er an der Frontwand (43) anschlägt und den radialen Kanal (46) freigibt.

8. Verfahren nach einem der vorherigen Ansprüche, dadurch gekennzeichnet, daß das Ende vor dem Spritzenkörper (101; 121; 1; 20; 42) umschlossen wird.

9. Vorgefüllte Injektionsvorrichtung, umfassend eine Injektionsnadel (116; 15; 23; 47), die in Kombination folgendes umfaßt:
- einen röhrenförmigen Spritzenkörper (101; 121; 1; 20; 42) aus einem Plastikmaterial, der an einem seiner Enden, hinteres Ende genannt, mit Fingergriffteilen (105; 5; 26; 53) und an seinem anderen Ende, vorderes Ende genannt, mit einem externen Sperrteil (104; 123; 7-9; 27-29; 54-56) versehen ist,
- eine röhrenförmige Hülse (109; 10; 31; 58) mit einem Innenquerschnitt, der so ausgestaltet ist, daß sie an dem Spritzenkörper (101; 121; 1; 20; 42) entlang gleiten kann, und die mit einem inneren Sperrteil (110, 111; 11; 32; 59) versehen ist, das mit dem des genannten Spritzenkörpers verbunden ist, wobei die genannte Hülse an einem ihrer Enden, vorderes Ende genannt, zwischen zwei Positionen gleiten kann;
- eine hintere Injektionsposition, in der einerseits das hintere Ende der Hülse (109; 10; 31; 58) an den Fingergriffteilen (105; 5; 26; 53) des Spritzenkörpers (101; 121; 1; 20; 42) anliegt und andererseits wenigstens das Ende vor dem Spritzenkörper (101; 121; 1; 20; 42) freigegeben ist,
- eine vordere Schutzposition nach dem Gebrauch, in der einerseits die jeweiligen Sperrteile (104; 110; 123, 110, 111; 7-9; 11; 27-29; 32; 54-56; 59) der Hülse (109; 10; 31; 58) und des Spritzenkörpers (101; 121; 1; 20; 42) sich im Zusammenwirkungszustand befinden und andererseits die Hülse das Ende vor dem Spritzenkörper verdeckt und in die Verlängerung des Letzteren verläuft,
- ein starres vorgefülltes Gefäß (114; 13; 34; 61), das ins Innere des Spritzenkörpers (101; 121; 1; 20; 42) eingeführt ist und einen Außenquerschnitt aufweist, der zum Innenquerschnitt des genannten Spritzenkörpers paßt,
dadurch gekennzeichnet, daß:
- die Handgriffteile (105; 5; 26; 53) und der Spritzenkörper ein einzelnes Teil bilden,
- der Körper der Spritze aus einem verformbaren Material besteht und das äußere Sperrteil (104; 123; 7-9; 27-29; 55-56) sich durch die radiale Verformung zwischen einer eingezogenen Position und einer ausgefahrenen Position verformt, und
- die Vorrichtung Sperrmittel (106; 6; 30; 57) gegen eine relative Verschiebung des starren Behältnises (114; 13; 34; 61) und des Spritzenkörpers (101; 121; 1; 20; 42) gegeneinander umfaßt.

10. Injektionsvorrichtung nach Anspruch 9, dadurch gekennzeichnet, daß die jeweiligen hinteren Enden der Hülse (109; 10; 31; 58) und des Spritzenkörpers (101; 121; 1; 20; 42) in der hinteren Injektionsposition dieses Letzteren durch Haltemittel fixiert sind, die unter der Wirkung der in Längsrichtung wirkenden Zugkraft nachgeben können, die auf die Hülse aufgebracht wird, um sie in ihre vordere Schutzposition nach dem Gebrauch zu verschieben.

11. Injektionsvorrichtung nach einem der Ansprüche 9 und 10, dadurch gekennzeichnet, daß der Spritzenkörper (121) ein Endstück des vorderen Endes (122) mit einem Querschnitt aufweist, der in Richtung des vorderen Endes des genannten Spritzenkörpers abnimmt, und auf dem das externe Sperrteil (123) angeordnet ist, wobei das genannte kegelstumpfförmige Endstück in Längsrichtung mit geschwächten Zonen (124) versehen ist, die deren radiales Ausfahren zulassen.

12. Injektionsvorrichtung nach einem der Ansprüche 9 und 10, dadurch gekennzeichnet, daß der Spritzenkörper (101; 1; 20; 42) eine Umfangswand aufweist, die mit wenigstens einem Längsschlitz (102; 2; 24; 51) versehen ist, der in der Höhe des äußeren Sperrmittels (104; 123; 7-9; 27-29; 54-56) verläuft, um die radialen Verformungen des genannten Sperrmittels zuzulassen.

13. Injektionsvorrichtung nach Anspruch 12, dadurch gekennzeichnet, daß der Spritzenkörper (101; 1; 20; 42) eine Umfangswand aufweist, die zwei Längsschlitze (102; 2; 24; 51) diametrisch gegenüberliegend aufweist.

14. Injektionsvorrichtung nach Anspruch 13, dadurch gekennzeichnet, daß der Spritzenkörper (101) einen Innendurchmesser aufweist, der im wesentlichen größer ist als der Außendurchmesser des starren Gefäßes (114), und innere Längsrippen (103) in der Höhe des inneren Sperrteils (104) des genannten Spritzenkörpers aufweist, die einen Durchmesser passend zum Außendurchmesser des starren Gefäßes (114) bildet.

15. Injektionsvorrichtung nach einem der Ansprüche 9 bis 14, dadurch gekennzeichnet, daß:
- das Sperrteil, das auf einem der Elemente, Spritzenkörper oder Hülse, angeordnet ist, die Form eines ringförmigen Vorsprungs hat, der eine ringförmige Rille (7; 92; 56) und Einkerbungen (110, 111; 9; 27; 28; 54, 55) aufweist, die auf beiden Seiten der Letzteren angeordnet sind,
- das Sperrteil, das auf dem anderen Element, d.h. der Hülse oder des Spritzenkörpers, angeordnet ist, von einer ringförmigen Rille (104; 123; 11; 32; 59) gebildet wird, die einen Abschnitt passend zum Außenabschnitt des hinteren Teils der Rille des Vorsprungs des ersten Elementes begrenzt.

16. Injektionsvorrichtung nach Anspruch 15, dadurch gekennzeichnet, daß die Einkerbungen (110) des Sperrmittels des einen der Elemente (109), Spritzenkörper oder Hülse, die sich am Ende des genannten Elementes befinden, eine Dicke aufweisen, die größer ist als die der zweiten Einkerbungen (111), die die Rille des genannten Sperrteils begrenzen.

17. Injektionsvorrichtung nach Anspruch 16, dadurch gekennzeichnet, daß die zweiten Einkerbungen (111) des Sperrmittels des Elementes (109), Spritzenkörper oder Hülse, eine Vorderfläche aufweisen, die sich über eine Sehne des genannten Elementes erstreckt und so ausgestaltet ist, daß in der Höhe der genannten Einkerbungen ein Umfang erreicht wird, der wenigstens so groß ist wie der des anderen Elementes (101) in der Höhe der ringförmigen Rille (104) des Letzteren.

18. Injektionsvorrichtung nach einem der Ansprüche 9 bis 17, dadurch gekennzeichet, daß:
- der Spritzenkörper (101; 1) ein vorderes Ende aufweist, das die Form einer Muffe (3) hat,
- das vorgefüllte starre Gefäß von einer vorgefüllten Spritze (114; 13) gebildet wird, die eine Injektionsnadel (116; 15) trägt, die einen vorderen Abschnitt mit einer Form aufweist, die in der Muffe (3) des Spritzenkörpers (101; 1) auf eine solche Weise aufgenommen werden kann, daß sich die Injektionsnadel (116; 15) in die Verlängerung des genannten Spritzenkörpers erstreckt.

19. Injektionsvorrichtung nach Anspruch 18, bei der die vorgefüllte Spritze (114; 13) ein hinteres Ende aufweist, das mit einem Fingergriffkragen (117; 16) versehen ist, dadurch gekennzeichnet, daß der Spritzenkörper (101; 1) in der Höhe seines hinteren Endes radiale Einkerbungen (106; 6) aufweist, die den Kragen (117; 16) aufnehmen und die Sperre gegen eine Verschiebung der vorgefüllten Spritze (114; 13) bewirken können.

20. Injektionsvorrichtung nach Anspruch 19, dadurch gekennzeichnet, daß die radialen Einkerbungen (106) in einem Ring (105) in der Form einer Kupelle mit einem Durchmesser angeordnet sind, der größer ist als der des Spritzenkörpers (101), und der in der hinteren Verlängerung des genannten Spritzenkörpers angeordnet ist, wobei der genannte Ring so ausgestaltet ist, daß er als vorderer Anschlag des hinteren Endes der Hülse (109) dient.

21. Injektionsvorrichtung nach Anspruch 20, dadurch gekennzeichnet, daß sie einen verformbaren Sprengring (107) aufweist, der so angeordnet ist, daß er von dem Kragen (117) der Spritze (114) auf eine solche Weise belastet wird, daß er eine spielfreie Sperre der Letzteren in den radialen Einkerbungen (106) gewährleistet.

22. Injektionsvorrichtung nach einem der Ansprüche 18 bis 21, in der die vorgefüllte Spritze (114; 13) von einer beweglichen Verschlußkappe (118; 17) verschlossen wird, die vor der Injektion die Injektionsnadel (116; 15) aufnimmt.

23. Injektionsvorrichtung nach einem der Ansprüche 9 bis 17, dadurch gekennzeichnet, daß:
- der Spritzenkörper (20) an seinem vorderen Ende mit einer Nase (21) versehen ist, die in Längsrichtung eine Bohrung (22) aufweist, die eine Injektionsnadel (23) auf eine solche Weise aufnimmt, daß Letztere ein aktives Injektionsendstück (23a) in der Verlängerung des genannten Spritzenkörpers und einen Durchbohrungsabschnitt (23b) bildet, der ins Innere desselben einmündet,
- das vorgefüllte starre Gefäß von einer Kartusche (34) gebildet wird, die mit einem Verschlußteil (35) versehen ist, das von dem Durchbohrungsendstück (23b) der Injektionsnadel (23) durchbohrt werden kann.

24. Injektionsvorrichtung nach Anspruch 23, dadurch gekennzeichnet, daß das Verschlußteil der Kartusche (34) aus einem Stopfen (35) besteht, der durchbohrt werden kann und in einer ersten Phase so angeordnet ist, daß er sich in einem Abstand vom Ende des Durchbohrungsendstückes (23b) der Injektionsnadel (23) befindet, wobei die genannte Kartusche an ihrem anderen Ende von einem zweiten Stopfen (37) in der Funktion eines Kolbens verschlossen wird.

25. Injektionsvorrichtung nach einem der Ansprüche 23 und 24, dadurch gekennzeichnet, daß der Spritzenkörper (20) in der Höhe seines hinteren Endes eine Innenrille (30) aufweist, die die Sperre gegen eine Verschiebung der Kartusche (34) gewährleistet, die in den genannten Spritzenkörper eingeführt wird.

26. Injektionsvorrichtung nach einem der Ansprüche 23 bis 25, dadurch gekennzeichnet, daß sie eine Verschlußkappe (39) aufweist, die das aktive Endstück (23a) der Injektionsspritze (23) aufnimmt, wobei das genannte Endstück und die Nase (21) des Spritzenkörpers (20) mit verbundenen Einklickteilen (40, 41) versehen sind, mit denen sie auf unbewegliche Weise fixiert werden können.

27. Injektionsvorrichtung nach einem der Ansprüche 9 bis 17, dadurch gekennzeichnet, daß:
- der Spritzenkörper (42) mit einem vorderen Ende versehen ist, das durch eine vordere Endwand (43) mit einer durch sie führenden Bohrung (44) verschlossen ist, und mit einer Längsbohrung (45) versehen ist, die in ihrer Umfangswand angeordnet ist und eine Injektionsnadel (47) auf solche Weise aufnimmt, das Letztere ein aktives Injektionsendstück (47a) in der Verlängerung der Frontwand (43) bildet, wobei die genannte Bohrung zwischen der genannten Frontwand und einem radialen Innenkanal (46) verläuft, der ins Innere des genannten Spritzenkörpers führt, wobei dieser radiale Kanal (46) in einem Abstand von der Frontwand (43) angeordnet ist und in Längsrichtung an einem Ansatz (49) im Inneren des Spritzenkörpers (42) anliegt, der eine Endkammer (50) begrenzt, die den genannten radialen Kanal aufweist, der einen kleineren Querschnitt aufweist als der laufende Innenquerschnitt des genannten Spritzenkörpers,
- das vorgefüllte starre Gefäß von einer Kartusche (61) mit einem Querschnitt gebildet wird, der so ausgestaltet ist, daß er am inneren Ansatz (49) des Spritzenkörpers (42) anliegt, wobei die genannte Kartusche an jedem ihrer Enden von einem Gleitstopfen (62, 63) verschlossen wird, die jeweils als vorderes und hinteres Ende bezeichnet werden, wobei der hintere Stopfen (62) als Kolben wirkt und der vordere Stopfen (63) eine solche Form aufweist, daß er in der Endkammer (50) des Spritzenkörpers (42) während der Betätigung des hinteren Stopfens (62) in einer Position aufgenommen werden kann, in der er an der Frontwand (43) anliegt und den Zugang am radialen Kanal (46) freigibt.

28. Injektionsvorrichtung nach Anspruch 27, dadurch gekennzeichnet, daß die Längsbohrung (45) einen inneren Zwischenansatz (48) als Anschlag für das hintere Ende der Injektionsnadel (47) bildet.

29. Injektionsvorrichtung nach einem der Ansprüche 27 und 28, dadurch gekennzeichnet, daß der Spritzenkörper (42) in der Höhe seines vorderen Endes eine Innenrille (57) aufweist, die die Sperrung der Verschiebung der Kartusche (61) gewährleistet, die in den genannten Spritzenkörper eingeführt wird.

30. Injektionsvorrichtung nach einem der Ansprüche 27 bis 29, dadurch gekennzeichnet, daß sie eine Verschlußkappe (64) aufweist, die das vordere Endstück des Spritzenkörpers (42) verdeckt und das aktive Endstück (47a) der Injektionsnadel (47) aufnimmt.
